(19) Europäisches Patentamt
European Patent Office
Office européen des brevets

(11) Publication number: 0 244 062 A2

(12) # EUROPEAN PATENT APPLICATION

(21) Application number: 87301770.1

(22) Date of filing: 27.02.87

(51) Int. Cl.$^3$: A 61 K 31/24
A 61 K 31/165, A 61 K 31/19
A 61 K 31/195, A 61 K 31/13-5
A 61 K 31/34, A 61 K 31/16
A 61 K 31/27, A 61 K 31/13
A 61 K 31/215, A 61 K 31/22

(30) Priority: 03.03.86 GB 8605160
30.05.86 GB 8613197

(43) Date of publication of application:
04.11.87 Bulletin 87/45

(84) Designated Contracting States:
BE CH DE ES FR GB GR IT LI LU NL SE

(71) Applicant: BEECHAM GROUP PLC
Beecham House Great West Road
Brentford Middlesex TW8 9BD(GB)

(72) Inventor: Arch, Jonathan Robert Sanders Beecham Pharm.
Great Burgh Yew Tree Bottom Road
Epsom Surrey KT18 5XQ(GB)

(72) Inventor: Fears, Robin Bradshaw Beecham Pharmaceuticals
Great Burgh Yew Tree Bottom Road
Epsom Surrey KT18 5XQ(GB)

(74) Representative: Jones, Pauline et al,
Beecham Pharmaceuticals Patents & Trade Marks Dept.
Great Burgh Yew Tree Bottom Road
Epsom Surrey KT18 5XQ(GB)

(54) **Use of aryl ethanol amine derivatives in the treatment of atherosclerosis.**

(57) A method for increasing high-density lipoprotein (HDL) cholesterol concentration and/or decreasing total cholesterol concentration and/or decreasing triglyceride concentration in human blood serum, which method comprises the administration of an effective, non-toxic amount of a compound selected from the list consisting of compounds of the, herein defined, formulae: (I), (II), (III), (IV), (V), (VI), (VII), (VIII), (IX), (X), (XI), (XII), (XIII), (XIV), (XV), (XVI), (XVII), (XVIII), (XIX), (XX), (XXI), (XXII), (XXIII), (XXIV), (XXV), (XXVI), (XXVII), (XXVIII), (XXIX) and (XXX); or, where appropriate, a pharmaceutically acceptable salt, ester or amide thereof.

EP 0 244 062 A2

Croydon Printing Company Ltd

TITLE MODIFIED
see front page

# NOVEL METHOD

The present invention relates to the use of certain ethanolamine derivatives in a method for increasing the high density-lipoprotein (HDL) cholesterol concentration and/or decreasing total cholesterol concentration and/or decreasing the triglyceride concentration in human blood serum.

European Patent Specification No. 0,006,735 discloses compounds of general formula (I):

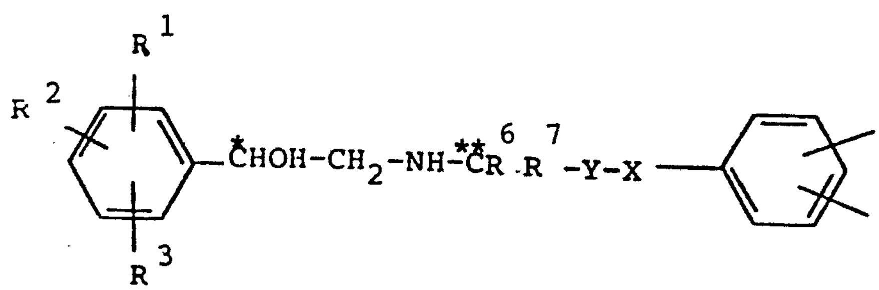

(I)

or a pharmaceutically acceptable salt thereof wherein $R^1$ is a hydrogen, fluorine or chlorine atom or a hydroxyl, hydroxymethyl, methyl, methoxyl, amino, formamido, acetamido, methylsulphonylamido, nitro, benzyloxy, methylsulphonylmethyl, ureido, trifluoromethyl or p-methoxybenzylamino group; $R^2$ is a hydrogen, fluorine or chlorine atom or a hydroxyl group; $R^3$ is a hydrogen or chlorine atom or a hydroxyl group; $R^4$ is a carboxylic acid group or a salt, ester or amide thereof; $R^5$ is a hydrogen, chlorine or fluorine atom or a methyl, methoxyl or hydroxyl group or a carboxylic acid group or a salt, ester or amide thereof; $R^6$ is a hydrogen atom or a methyl, ethyl or propyl group; $R^7$ is a hydrogen atom or a methyl, ethyl or propyl group; X is an oxygen atom or a bond; and Y

is an alkylene group of up to 6 carbon atoms or a bond; which compounds show anti-obesity and/or anti-hyperglycaemic activity.

European Patent Specification No. 0,023,385 discloses compounds of a general formula (II):

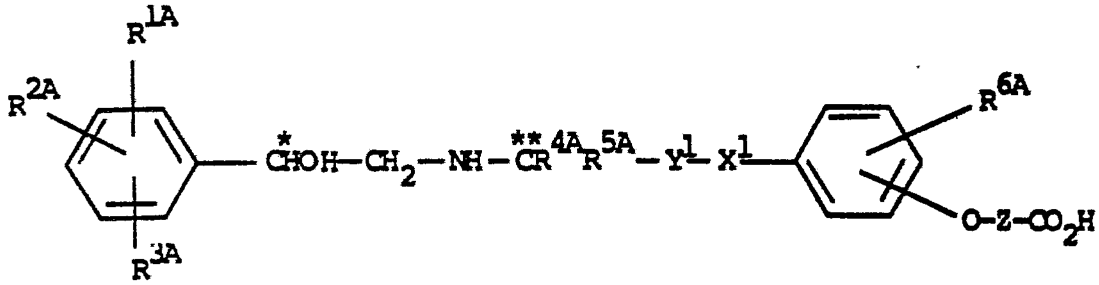

(II

or a pharmaceutically acceptable salt, ester or amide thereof wherein $R^{1A}$ is a hydrogen, fluorine or chlorine atom or a hydroxyl, hydroxymethyl, methyl, methoxyl, amino, formamido, acetamido, methylsulphonylamido, nitro, benzyloxy, methylsulphonylmethyl, ureido, trifluoromethyl or p-methoxybenzylamino group; $R^{2A}$ is a hydrogen, fluorine or chlorine atom or a hydroxyl group; $R^{3A}$ is a hydrogen or chlorine atom or a hydroxyl group; or $R^{1A}$, $R^{2A}$, and $R^{3A}$ each represents a bromine atom; $R^{4A}$ is a hydrogen atom or a methyl group; $R^{5A}$ is a hydrogen atom or a methyl group; $R^{6A}$ is a hydrogen, fluorine or chlorine atom or a methyl, methoxyl or hydroxy group; $X^1$ is an oxygen atom or a bond; $Y^1$ is an alkylene group of up to 6 carbon atoms or a bond; and Z is an alkylene, alkenylene or alkynylene group of up to 10 carbon atoms; which compounds show anti-obesity and/or anti-hyperglycaemic activity.

European Patent Specification No. 0,028,105 discloses a particular group of compounds, falling within formula (I), of formula (III):

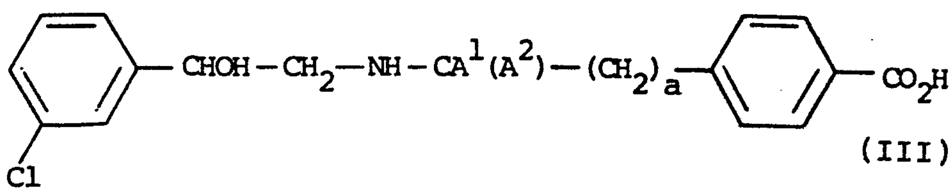

(III)

and esters, amides and pharmaceutically acceptable salts thereof wherein $A^1$ is a hydrogen atom or a methyl group and $A^2$ is a hydrogen atom or a methyl group and a is 1,2 or 3.

The compounds of formula (III) are described as having good anti-obesity and anti-hyperglycaemic properties coupled with low side effects.

European Patent Specification No. 0,040,915 also discloses a particular group of compounds of formula (IV)

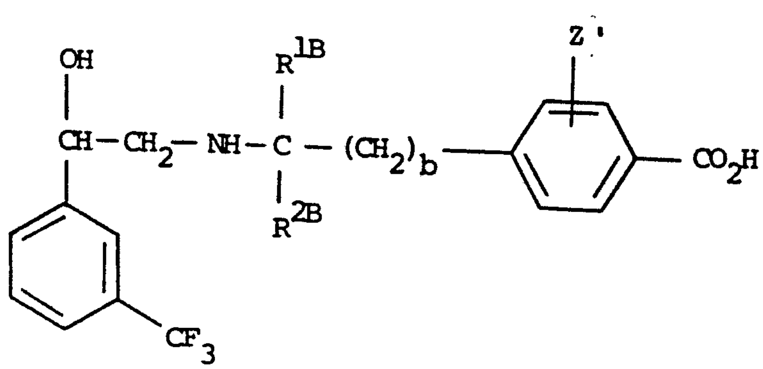

(IV)

or a pharmaceutically acceptable salt, lower alkyl or aralkyl ester or amide thereof, wherein $R^{1B}$ and $R^{2B}$ which may be the same of different, are each a hydrogen atom or a methyl group, b is 1,2 or 3, and $Z^1$ is a $C_{1-6}$ alkyl or $C_{1-6}$ alkoxy group, or a halogen or hydrogen atom.

The compounds of formula (IV) are described as having anti-obesity and/or hypoglycaemic activity coupled with low cardiac stimulant activity. Particular compounds of formula (IV) also have anti-inflammatory activity.

European Patent Specification No. 0,040,000 discloses compounds of formula (V):

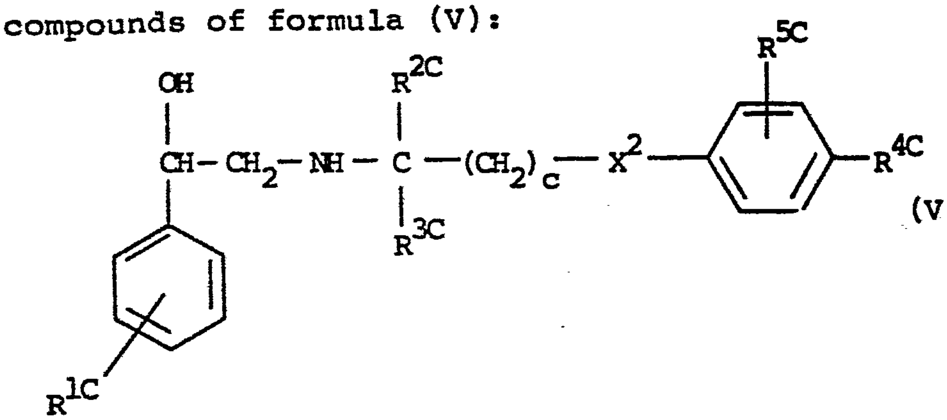

(V)

or a pharmaceutically acceptable salt thereof, in which $X^2$ is an oxygen atom or a bond, $R^{1C}$ is a hydrogen, fluorine, chlorine or bromine atom or a trifluoromethyl or $C_{1-4}$ alkyl group, each of $R^{2C}$ and $R^{3C}$ is a hydrogen atom or a $C_{1-4}$ alkyl group, $R^{4C}$ is a $C_{1-4}$ alkyl group, $R^{5C}$ is a hydrogen atom or a $C_{1-4}$ alkyl group and c is an integer of from 1 to 3.

The compounds of formula (V) are described as having anti-obesity and/or hypoglycaemic activity coupled with low cardiac stimulant activity. Derivatives of compound (V) are stated to have topical anti-inflammatory activity.

European Published Application No. 0,052,963 discloses compounds of formula (VI):

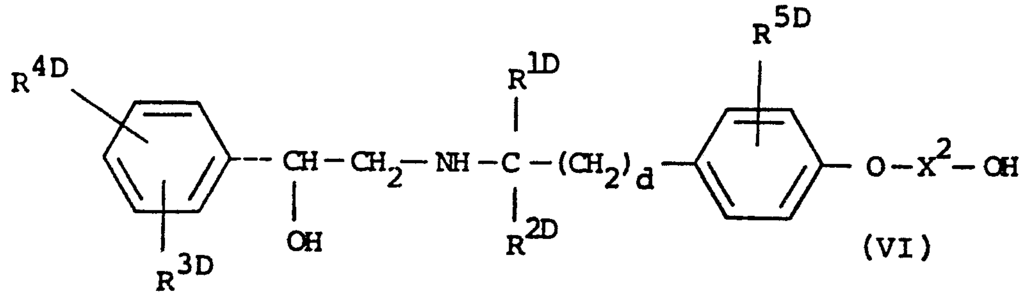

(VI)

or a salt thereof; wherein $R^{1D}$ is hydrogen, methyl or ethyl; $R^{2D}$ is hydrogen, methyl or ethyl; $R^{3D}$ is hydrogen, fluorine, chlorine, bromine or trifluoromethyl; each of $R^{4D}$ and $R^{5D}$ is hydrogen, fluorine, chlorine, bromine, $C_{1-6}$ alkyl or branched alkylene; d is 1 or 2; and $X^2$ is $C_{1-12}$ straight or branched alkylene.

The compounds of formula (VI) are described as having anti-obesity and/or hypoglycaemic and and/or anti-inflammatory and/or platelet aggregation inhibition activity coupled with low cardiac stimulant activity for particular compounds of formula (VI).

European Patent Specification No. 0,066,351 discloses compounds of formula (VII):

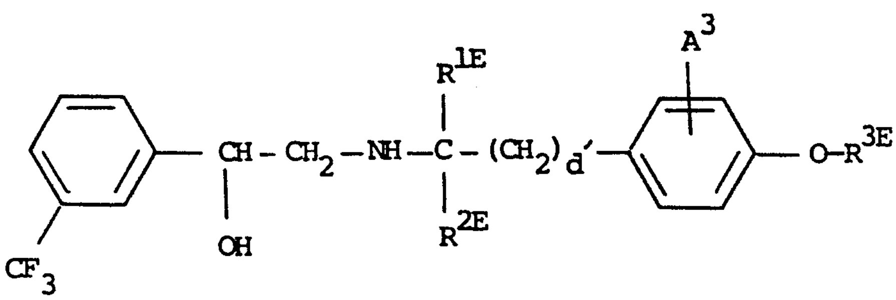

(VII)

or a salt thereof;

wherein

$R^{1E}$ is hydrogen or methyl;

$R^{2E}$ is hydrogen or methyl;

d' is 1 or 2;

$R^{3E}$ is hydrogen, $C_{1-12}$ alkyl, $C_{1-10}$ cycloalkyl or benzyl optionally substituted with $C_{1-6}$ alkyl, $C_{1-6}$ alkoxy, or halogen;

and

$A^3$ is hydrogen, halogen, hydroxy, $C_{1-6}$ alkyl or $C_{1-6}$ alkoxy.

The compounds of formula (VII) are described as having anti-hyperglycaemic and/or anti-obesity and/or anti-inflammatory and/or platelet aggregation inhibition activity.

European Patent Specification No. 0,061,907 discloses compounds of formula (VIII):

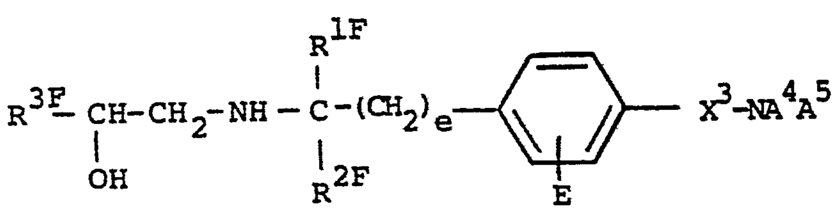

(VIII):

and salts thereof

wherein

$R^{1F}$ is hydrogen or methyl;

$R^{2F}$ is hydrogen or methyl;

$R^{3F}$ is phenyl optionally substituted with one or two of the following: fluorine, chlorine, bromine, trifluoromethyl or $C_{1-6}$ alkyl, or is benzofuran-2-yl;

e is 1 or 2;

$X^3$ is a $C_{1-15}$ straight or branched alkylene group;
$A^4$ is hydrogen, $C_{1-6}$ alkyl, or optionally substituted benzyl;
$A^5$ is hydrogen, $C_{1-6}$ alkyl, or optionally substituted benzyl; and
E is hydrogen, halogen, $C_{1-6}$ alkoxy, $C_{1-6}$ alkyl, or mono- or di-$C_{1-6}$ alkylamino.

The compounds of formula (VIII) are described as having anti-obesity and/or anti hyperglycaemic and/or anti-inflammatory and/or platelet aggregation inhibition activity, coupled with low cardiac stimulant activity for certain compounds of formula (VIII).

European Published Patent Application No.0,070,133 discloses compounds of formula (IX):

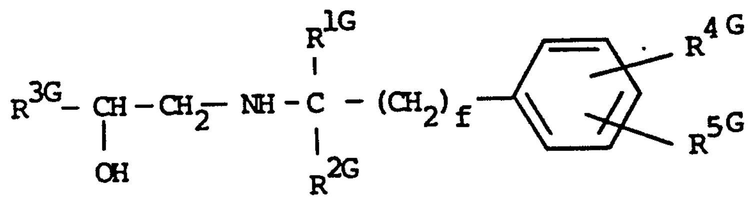

(IX)

and salts and <u>in vivo</u> hydrolysable acyl derivatives thereof, wherein

$R^{1G}$ is hydrogen or methyl;
$R^{2G}$ is hydrogen or methyl;
$R^{3G}$ is phenyl unsubstituted or substituted with one or two of the following:- fluorine, chlorine, bromine, trifluoromethyl, $C_{1-6}$ straight or branched chain alkyl or $C_{1-6}$ straight or branched chain alkoxy; or is benzofuran-2-yl;
$R^{5G}$ is a hydrogen, halogen, hydroxy, lower alkyl or lower alkoxy;
$R^{4G}$ is a moiety

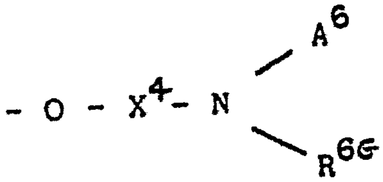 or $- O - X^5 - CN$

wherein $X^4$ is a $C_{2-10}$ alkylene group which may be straight or branched, provided that the oxygen and nitrogen are separated by at least two carbon atoms,

$X^5$ is a $C_{1-9}$ alkylene group which may be straight or branched,

$A^6$ is hydrogen; a $C_{1-6}$ straight or branched alkyl group or benzyl optionally substituted with halogen, lower alkyl or lower alkoxy,

$R^{6G}$ is hydrogen; a $C_{1-6}$ straight or branched alkylgroup; a $C_{1-6}$ straight or branched alkysulphonyl group or

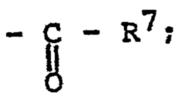

$R^{7G}$ is a $C_{1-6}$ straight or branched alkyl group; $C_{1-6}$ straight or branched alkoxy; amino, mono- or di- ($C_{1-6}$ straight or branched chain alkyl)amino;

or 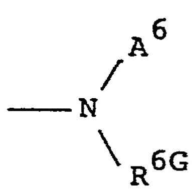 is a 5,6 or 7 membered cyclic amino group

optionally containing a second hetero atom selected from oxygen, nitrogen and sulphur,

and f is 1 or 2

The compounds of formula (IX) are described as having anti-obesity and/or anti-hyperglycaemic and/or anti-inflammatory and/or platelet aggregation inhibition activity, coupled with low cardiac stimulant activity for particular compounds of formula (IX)

European Patent Specification No. 0,070,134 and United Kingdom Patent Specification No. 1,574,208 disclose compounds of formula (X):

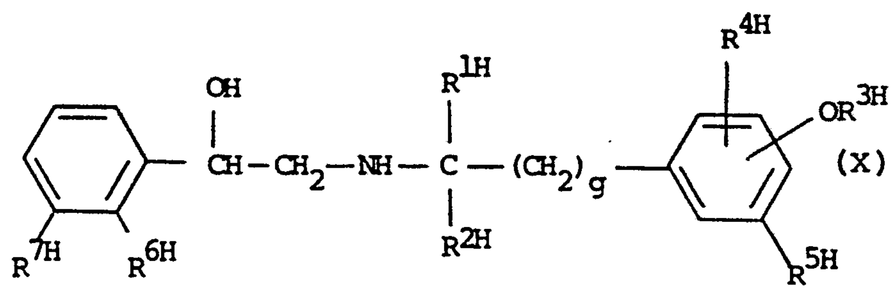

(X)

or a salt thereof;

wherein

$R^{1H}$ is hydrogen or methyl,

$R^{2H}$ is hydrogen or methyl,

$R^{3H}$ is hydrogen, $C_{1-12}$ straight or branched alkyl, $C_{3-10}$ cycloalkyl, phenyl $(C_{1-4})$alkyl or benzyl optionally substituted by $C_{1-4}$ alkyl, $C_{1-4}$ alkoxy or halogen;

$R^{4H}$ is hydrogen, halogen, hydroxy, $C_{1-4}$ alkyl or $C_{1-4}$ alkoxy,

$R^{5H}$ is hydrogen or fluorine,

$R^{6H}$ is hydrogen or fluorine,

$R^{7H}$ is halogen;

and g is 1 or 2

The compounds of formula (X) have anti-hyperglycaemic and anti-obesity activity.

European Patent Specification No. 0,095,827 discloses compounds of formula (XI):

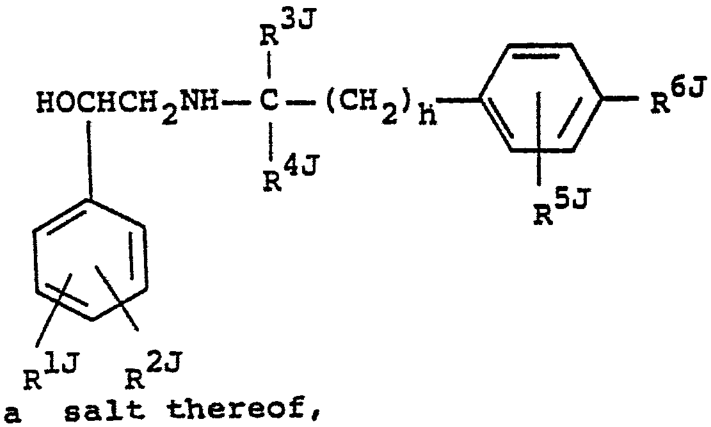

(XI)

or a salt thereof,

wherein

$R^{1J}$ is hydrogen, halogen or trifluoromethyl,

$R^{2J}$ is hydrogen or halogen,

$R^{3J}$ is hydrogen or methyl,

$R^{4J}$ is hydrogen or methyl,

$R^{5J}$ is hydrogen or halogen,

$R^6$ is 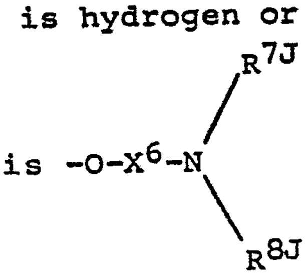

$X^6$ is a $C_{3-20}$ straight or branched alkylene group having at least two carbon stoms interposed between the oxygen and nitrogen atoms and having a hydroxyl substituent on a carbon atom not directly bonded to the oxygen or nitrogen atoms;

$R^{7J}$ is hydrogen, a $C_{1-6}$ straight or branched chain alkyl group, or optionally substituted benzyl, $R^{8J}$ is hydrogen, a $C_{1-6}$ straight or branched chain alkyl group, or optionally substituted benzyl,
or

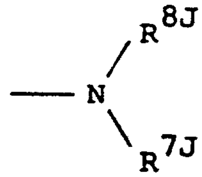

is a 5,6 or 7 membered, saturated heterocyclic group containing a total of one or two heteroatoms, the second hetero-atom, when present, being selected from nitrogen, oxygen and sulphur,
and h is 1 or 2.

The compounds of formula (XI) are described as having anti-hyperglycaemic and/or anti-obesity activity, coupled with low cardiac stimulant activity for particular compounds of formula (XI).

European Patent Application No. 0,099,707 discloses compounds of formula (XII):

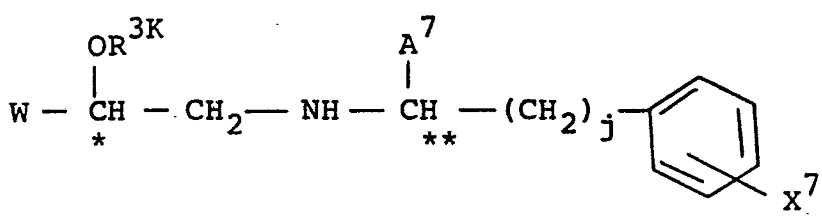

(XII)

or a pharmaceutically acceptable salt, ester or amide thereof, in which:

W is an optionally substituted phenyl group of the formula:

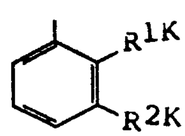

wherein $R^{1K}$ is hydrogen or fluorine,

$R^{2K}$ is hydrogen, $C_{1-6}$ alkyl, halogen or trifluoromethyl;

or W is a phenoxymethyl or benzofuran-2-yl group;

$R^{3K}$ is $C_{1-12}$ alkyl or phenyl-$(C_{1-6})$-alkyl;

$A^7$ is hydrogen or methyl,

$X^7$ is carboxy, $-Z^2-CO_2H$, $-Z^2-OH$, $T-Z^2-CO_2H$, $-Z^2-NR^{4K}R^{5K}$, $-T-Y^2-OM$, $-T-Y^2-NR^{4K}R^{5K}$, or $-T-R^{6K}$, in the para- or meta-position with respect to the $-(CH_2)_j$ group,

wherein

$R^{4K}$ and $R^{5K}$ are each hydrogen or $C_{1-6}$ alkyl,

$R^{6K}$ is $C_{1-6}$ alkyl,

T is O, S, -NH or $-N-R^{7K}$, in which $R^{7K}$ is $C_{1-6}$ alkyl,

$Z^2$ is a $C_{1-10}$ straight or branched alkylene group optionally containing a carbon-carbon double bond;

$Y^2$ is a $C_{2-10}$ straight or branched alkylene group, provided that the hetero atoms in $-T-Y^2-OM$ and $-T-Y^2NR^{4K}R^{5K}$ are separated by at least two carbon atoms,

M is hydrogen, $C_{1-6}$ alkyl or phenyl and j is 1 or 2.

The compounds of formula (XII) are disclosed as having anti-hyperglycaemic and/or anti-obesity and/or anti-inflammatory and/or platelet aggregation inhibition activity coupled with low cardiac stimulant activity.

European Published Patent Application No.0,139,921 discloses compounds of formula (XIII):

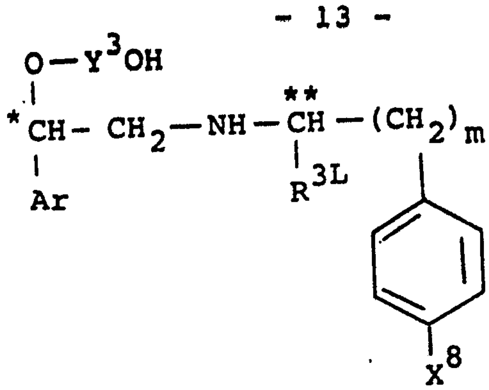

(XIII)

or a pharmaceutically acceptable salt, ester or amide thereof, wherein:

Ar is benzofuran-2-yl, or phenyl optionally substituted by groups $R^{1L}$ and/or $R^{2L}$ wherein $R^{1L}$ is halogen, trifluoromethyl or hydroxy and $R^{2L}$ is halogen; $R^{3L}$ is hydrogen or methyl; and $X^8$ is $-O(CH_2)_kCO_2H$, $(CH_2)$ $M^1$ or $-CO_2H$

in which

k is an integer from 1 to 6,

l is an integer from 2 to 7,

$M^1$ is hydroxy, $C_{1-6}$ alkoxy, phenyl $C_{1-6}$ alkoxy, or $-NR^{4L}$ $R^{5L}$ in which $R^{4L}$ and $R^{5L}$ are each hydrogen or $C_{1-6}$ alkyl or together form a five or six membered ring; $Y^3$ is $C_{2-6}$ straight or branched alkylene, with at least two carbon atoms between the -O- and -OH; and m is 1 or 2.

The compounds of formula (XIII) are described as having anti-obesity and/or anti-hyperglycaemic activity.

European Published Patent Application No. 0,142,102 discloses compounds of formula (XIV):

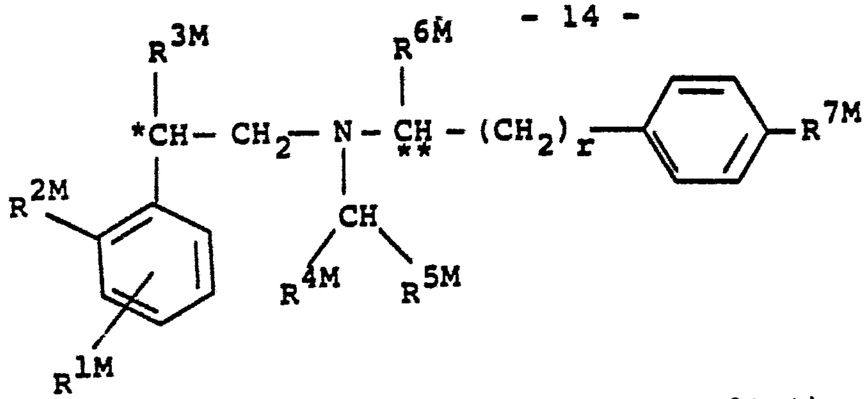

(XIV)

or a pharmaceutically acceptable salt thereof,

in which

$R^{1M}$ is hydrogen, halogen or trifluoromethyl;
$R^{2M}$ is hydrogen or halogen;

$R^{3M}$ is hydroxyl, $C_{1-6}$ alkoxy or $-N\langle^{R^{8M}}_{R^{9M}}$ where

$R^{8M}$ and $R^{9M}$ are each hydrogen or $C_{1-6}$ alkyl; $R^{4M}$ is hydrogen or $C_{1-6}$ alkyl, $R^{5M}$ is hydrogen, $C_{1-6}$ alkyl, $C_{1-6}$ alkyl optionally substituted by hydroxy; cyano, $C_{1-6}$ alkenyl or $C_{1-6}$ alkynyl optionally substituted by carboxy or esters and amides thereof,

phenyl, $C_{1-6}$ alkyl phenyl or a group$-(CH_2)_nN\langle^{R^{12M}}_{R^{13M}}$

wherein $R^{12M}$ and $R^{13M}$ are each hydrogen or $C_{1-6}$ alkyl or together, along with the nitrogen to which they are attached, form a 5- or 6- membered ring, and n is 1 or 2; $R^{6M}$ is hydrogen or methyl: $R^{7M}$ is $-O(CH_2)_pCO_2H$, $-O(CH_2)_qM^2$, $-CO_2H$; or an ester or amide thereof in which

p is an integer from 1 to 6
q is an integer from 2 to 7, and

$M^2$ is hydroxy, $C_{1-6}$ alkoxy or 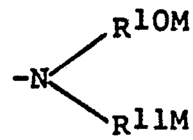

in which
$R^{10M}$ and $R^{11M}$ are each hydrogen or $C_{1-6}$ alkyl

or 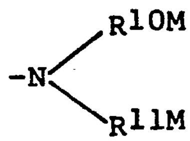 together form a five or six membered ring; and r is 1 or 2; with the proviso that when n is 2 and $R^{1M}$, $R^{2M}$, $R^{4M}$ and $R^{6M}$ are each hydrogen and $R^{3M}$ is hydroxyl, $R^{5M}$ is not hydroxymethyl or 1-hydroxy ethyl when $R^{7M}$ is $CONH_2$.

The compounds of EP142102A are described as having anti-hyperglycaemic and/or anti-obesity activity.

European Published Patent Application No.0,164,700 discloses compounds of formula (XV):

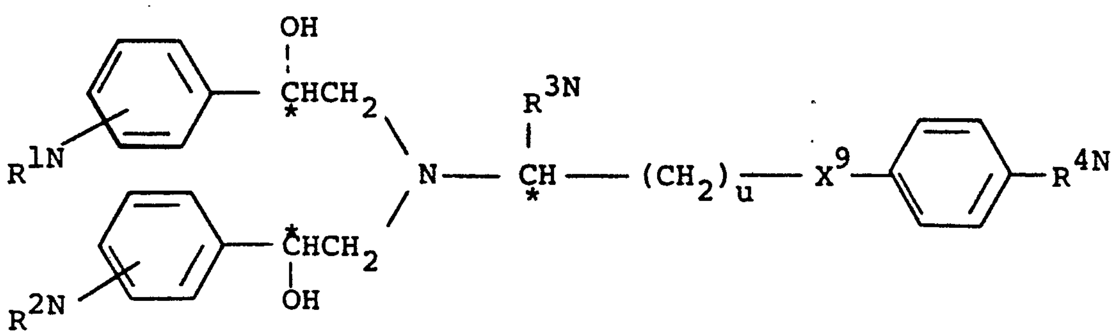

(XV)

or a pharmaceutically acceptable salt thereof, in which each or $R^{1N}$ and $R^{2N}$, which may be the same or different, is hydrogen, halogen, or trifluoromethyl, $R^{3N}$ is hydrogen or methyl,

$R^{4N}$ is $-O(CH_2)_s\ CO_2H$, $-O(CH_2)_t-OH$ or $-O(CH_2)_t$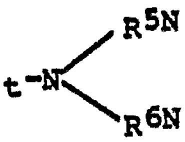

or an ester or amide derivative thereof, in which

s is an integer from 1 to 6
t is an integer from 2 to 7
$R^{5N}$ and $R^{6N}$ are each hydrogen or $C_{1-6}$ alkyl,

or - 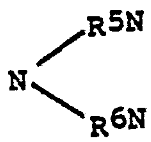 together form a five or six membered ring,
$X^9$ is -O- or a bond,
and u is 1 or 2.

The compounds of EP164700A are described as having β-agonist activity, being useful as anti-obesity and/or anti-hyperglycaemic agents.

European Published Patent Application No. 0,170,121 discloses compounds of formula (XVI):

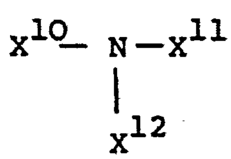

(XVI)

wherein $X^{10}$ represents a 2-hydroxy-3-aryloxypropyl radical unsubstituted or substituted in the aryl moiety or a 2-hydroxy-2-benzofuranylethyl group; and

$X^{11}$ represents a 2-hydroxy-3-aryloxypropyl or 2-hydroxy-2-arylethyl radical unsubstituted or

substituted in the aryl moiety or a 2-hydroxy-2-benzofuranylethyl group; and

$X^{12}$ represents a 2- or 3-arylpropyl or 2-aryloxyethyl radical substituted in the aryl moiety and unsubstituted or methyl-substituted in the alkyl chain;

or a compound of formula (XVI) wherein one of the -CHOH- moieties in any one of the 2-hydroxyethyl or 2-hydroxypropyl moieties is present as a carbonyl group and/or a compound of formula (XVI) wherein up to two of the aforementioned -CHOH- moieties may be present in the form of a pharmaceutically acceptable in-vivo hydrolysable ester; or a pharmaceutically acceptable salt thereof.

The compounds of formula (XVI) are described as having anti-hyperglycaemic and/or anti-obesity activity.

European Published Patent Application No. 0,171,702 discloses compounds of formula (XVII):

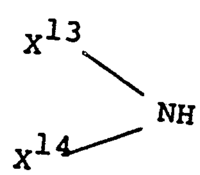

(XVII)

or a pharmaceutically acceptable salt thereof, wherein:

$X^{13}$ represents a 2-hydroxy-3-phenoxypropyl radical unsubstituted or substituted in the phenyl ring, or a 2-hydroxy-2-phenethyl radical unsubstituted or substituted in the phenyl ring or a 2-hydroxy-2-(benzofuranyl)ethyl radical or a

2-hydroxy-3-(indolyl)oxypropyl radical wherein a phenyl moiety in either of the benzofuranyl and indolyl moieties may be substituted or unsubstitued, and

$X^{14}$ represents a benzoxazinonylethyl, benzoxazinonylpropyl or benzoxazinonyloxyethyl radical unsubstituted or substituted in the benzoxazinonyl radical and/or lower alkyl-substituted in the alkyl chain.

The compounds of formula (XVII) are described as having anti-obesity and/or anti-hyperglcaemic and/or positive inotropic activity.

European Patent Specification No. 0,029,320 discloses compounds of formula (XVIII):

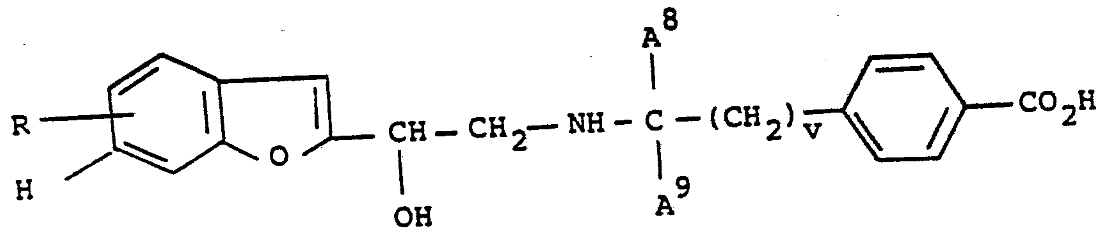

(XVIII)

and esters, amides, or pharmaceutically acceptable salts thereof, wherein $A^8$ is hydrogen or methyl; $A^9$ is hydrogen or methyl; v is 1,2 or 3; and R is hydrogen, chlorine, bromine, hydroxy, methoxy, nitro, amino or trifluoromethyl.

The compounds of EP29320 are described as having good anti-obesity and/or anti-hyperglycaemic activity coupled with a low level of side effects.

European Published Patent Application No.0,051,917 discloses compounds of formula (XIX):

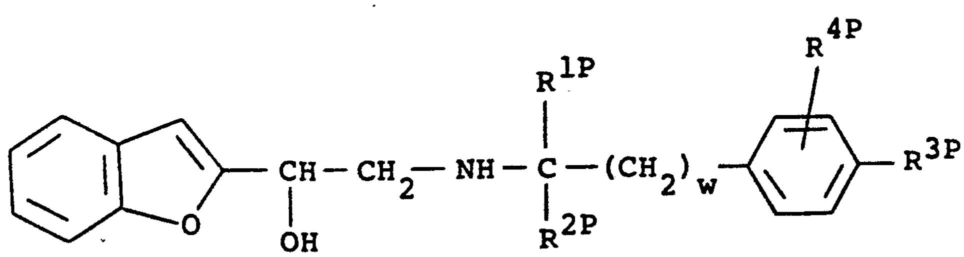

(XIX)

or an ester, amide or salt thereof, where appropiate, wherein

$R^{1P}$ is hydrogen or methyl;
$R^{2P}$ is hydrogen or methyl;
$R^{3P}$ is hydrogen, hydroxy, ($C_{1-6}$) alkoxy, benzyloxy or a group $X^{15}$-$Y^4$-$Z^3$, wherein

(i) $X^{15}$ is a bond or oxygen, $Y^4$ is $C_{1-6}$ straight or branched alkylene, and $Z^3$ is hydrogen or carboxy;
or
(ii) $X^{15}$ is a bond or moiety $-O-CH_2-$, $Y^4$ is $C_{2-6}$ straight or branched alkenylene and $Z^3$ is carboxy;

$R^{4P}$ is selected from hydrogen, hydroxy, halogen, $C_{1-6}$ alkyl and $C_{1-6}$ alkoxy; and w is 1,2 or 3.

The compounds of EP51917A are described as having anti-obesity, hypoglycaemic, anti-inflammatory and platelet-aggregation inhibition activity.

European Published Patent Application No. 0,140,359 discloses compounds of formula (XX):

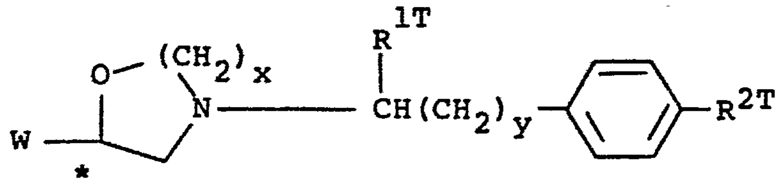 (XX)

or a salt thereof, in which

W is phenyl optionally substituted by halogen or trifluoromethyl, or a benzofuran-2-yl group,
$R^{1T}$ is hydrogen or methyl,
$R^{2T}$ is carboxyl or a group $O-Z^4-CO_2H$ or an ester or amide thereof; a group $O-E^1-NR^{3T}$ or a group $O-E^1-OR^{5T}$,
wherein
$R^{3T}$, $R^{4T}$ and $R^{5T}$ each represents hydrogen or $C_{1-6}$ alkyl, $Z^4$ is a $C_{1-6}$ straight or branched alkylene chain, x is 2 or 3, y is 1 or 2, and $E^1$ is $C_{2-7}$ straight or branched alkylene chain with at least two carbon atoms separating the two heteroatoms in the group $R^{2T}$.

The compounds of EP140359A are described as having anti-hyperglycaemic and/or anti-obesity activity.

European Published Patent Application No. 0,171,519 discloses compounds of formula (XXI):

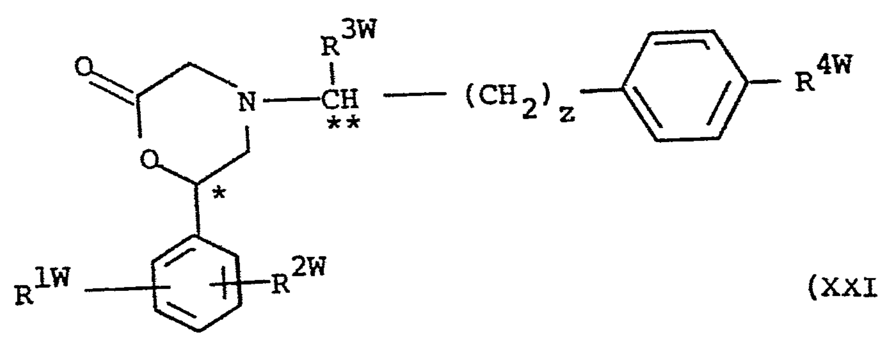

(XXI)

or a pharmaceutically acceptable salt, thereof, in which

$R^{1W}$ is hydrogen, halogen or trifluoromethyl,

$R^{2W}$ is hydrogen or halogen,

$R^{3W}$ is hydrogen or methyl,

$R^{4W}$ is $-O(CH_2)_\alpha\ CO_2H$, $-O(CH_2)_\beta M^3$, $-CO_2H$ or an ester or amide derivative thereof

in which

a' is an integer from 1 to 6,

b' is an integer from 2 to 7, and

$M^3$ is hydroxy, $C_{1-6}$ alkoxy or 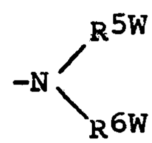

in which

$R^{5W}$ and $R^{6W}$ are each hydrogen or $C_{1-6}$ alkyl

or 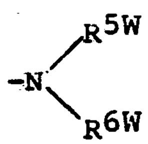 together form a five or six membered ring, and

z is 1 or 2.

The compounds of formula (XXI) are described as having β-agonist activity and are useful as anti-obesity and/or anti-hyperglycaemic agents.

European Published Patent Application No. 0,170,135 discloses compounds of formula (XXII):

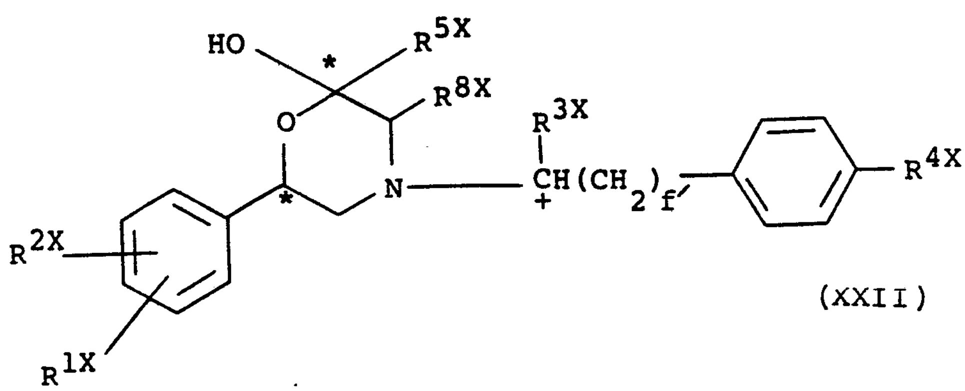

(XXII)

or a pharmaceutically acceptable salt thereof,
in which

$R^{1X}$ is hydrogen, halogen, or trifluoromethyl;

$R^{2X}$ is hydrogen or halogen;

$R^{3X}$ is hydrogen or methyl;

$R^{4X}$ is $-O(CH_2)_{d'}$ $CO_2H$ or an ester or amide derivative thereof, $O(CH_2)_{e'}$ $M^4$ or $-CO_2H$ or an ester or amide derivative thereof

wherein

d′ is an integer from 1 to 6,

e′ is an integer from 2 to 7, and

$M^4$ is hydroxy, $C_{1-6}$ alkoxy or 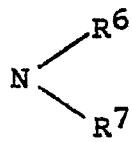

wherein

$R^{6X}$ and $R^{7X}$ are each hydrogen or $C_{1-6}$ alkyl

or 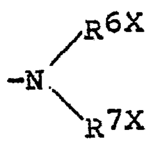 together form a five or six membered ring;

$R^{5X}$ is $C_{1-6}$ alkyl; $C_{1-6}$ alkyl substituted by carboxy or esters and amides thereof; or phenyl optionally substitued by $C_{1-6}$ alkyl, halogen, alkoxy or trifluoromethyl;

R8X is hydrogen or $C_{1-6}$ alkyl or $R^{8X}$ together with R5X form a carbocyclic ring; and
f ′is 1 or 2

The compounds of formula (XXII) are described as having anti-hyperglycaemic and/or anti-obesity activity. European Patent Specification No.0,021,636 discloses compounds of formula (XXIII):

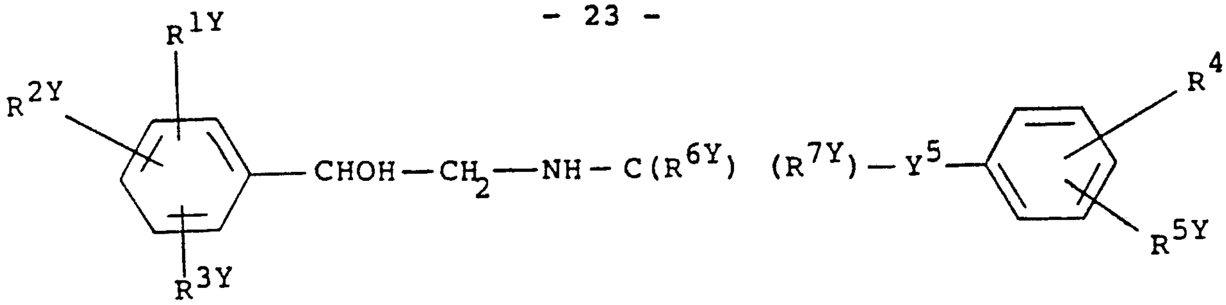

(XXIII)

or a pharmaceutically acceptable salt thereof, wherein $R^{1Y}$ is a hydrogen, fluorine, or chlorine atom or a hydroxyl, hydroxymethyl, methyl, methoxyl, amino, formamido, acetamido, methylsulphonylamido, nitro, benzyloxy, methylsulphonylmethyl, ureido, trifluoromethyl or p-methoxybenzylamino; $R^{2Y}$ is a hydrogen, fluorine or chlorine atom or a hydroxyl group; $R^{3Y}$ is a hydrogen or chlorine atom or a hydroxyl group; or $R^{1Y}$, $R^{2Y}$ and $R^{3Y}$ each independently represents a bromine atom; $R^{4Y}$ is an alkyl group of 1 to 10 carbon atoms substituted by a hydroxyl, lower alkoxyl or $OCH_2CO_2H$ group or lower alkyl ester thereof; $R^{5Y}$ is a hydrogen, chlorine or fluorine atom or a methyl, methoxyl or hydroxyl group or a carboxylic acid group or a salt, ester or amide thereof; $R^{6Y}$ is a hydrogen atom or a methyl, ethyl or propyl group; $R^{7Y}$ is a hydrogen atom or a methyl, ethyl or propyl group; and $Y^5$ is an alkylene group of up to 6 carbon atoms or a bond.

The compounds of EP21636 are described as having anti-obesity properties and/or anti-hyperglycaemic properties.

European Patent Specification No. 0,025,331 discloses compounds of formula (XXIV):

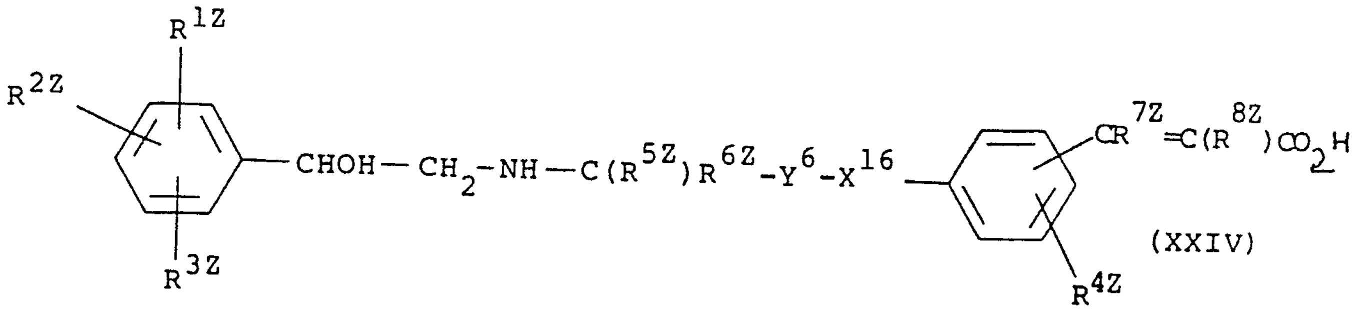

(XXIV)

or a pharmaceutically acceptable ester, amide or salt thereof wherein $R^{1Z}$ is a hydrogen, fluorine or chlorine atom or a hydroxyl, hydroxymethyl, methyl, methoxyl, amino, formamido, acetamido, methylsulphonylamido, nitro, benzyloxy, methylsulphonylmethyl, ureido, trifluoromethyl or p-methoxybenzylamino, $R^{2Z}$ is a hydrogen, fluorine or chlorine atom or a hydroxyl group; $R^{3Z}$ a hydrogen or chlorine atom or a hydroxyl group; or $R^{1Z}$, $R^{2Z}$ and $R^{3Z}$ each independently represents a bromine atom; $R^{4Z}$ is a hydrogen, chlorine or fluorine atom or a methyl, methoxyl or hydroxy group or a carboxylic acid group or a salt, ester or amide thereof; $R^{5Z}$ is a hydrogen atom or a methyl group; $R^{6Z}$ is a hydrogen atom or a methyl group; $R^{7Z}$ is a hydrogen atom or a methyl or ethyl group; $R^{8Z}$ is a hydrogen atom or a methyl or ethyl group; $X^{16}$ is an oxygen atom or a bond; and $Y^6$ is an alkylene group of up to 5 carbon atoms or a bond.

The compounds of EP25331 are described as being useful for the treatment of obesity or hyperglycaemia.

European Published Application No. 0,089,154 discloses _inter alia_ compounds of formula (XXV):

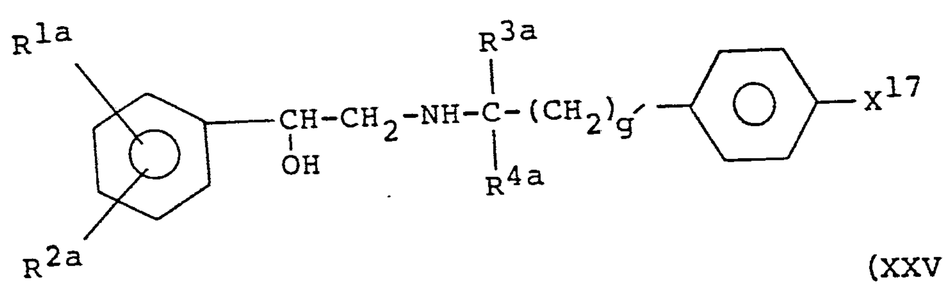

(XXV)

or a pharmaceutically acceptable salt thereof, wherein;

$R^{1a}$ is hydrogen, halogen or trifluoromethyl,
$R^{2a}$ is hydrogen or halogen,
$R^{3a}$ is hydrogen or methyl,
$R^{4a}$ is hydrogen or methyl,
g′ is 1,

$X^{17}$ is $-Y^7-SR^{5a}$, $-Y^7-\underset{O}{\underset{\|}{S}}-R^{5a}$ or $-Y^7-\overset{O}{\overset{\|}{\underset{O}{\underset{\|}{S}}}}-R^{5a}$

$R^{5a}$ is optionally substituted benzyl or $C_{1-12}$ straight or branched alkyl optionally substituted by carboxy, $C_{1-6}$ alkoxycarbonyl, benzyloxycarbonyl, aminocarbonyl, mono- or di-$(C_{1-6})$alkylamino-carbonyl, hydroxy, $C_{1-6}$ alkoxy, benzyloxy, phenoxy, amino, mono- or di-$(C_{1-6})$alkylamino, $C_{1-6}$ alkylthio, benzylthio, or phenylthio

provided that, when $R^{5a}$ is alkyl substituted by hydroxy, alkoxy, alkylthio, phenylthio, benzylthio, amino, alkylamino or benzyloxy, such substituents are separated from the sulphur atom by at least two carbon atoms, and

$Y^7$ is methylene or a bond.
The compounds of EP89154A are described as having anti-obesity and/or anti-hyperglycaemic and/or anti-inflammatory activity, this activity being coupled with low cardiac stimulant activity for particular members of the class.

European Published Patent Application No. 0,091,749 discloses compounds of formula (XXVI):

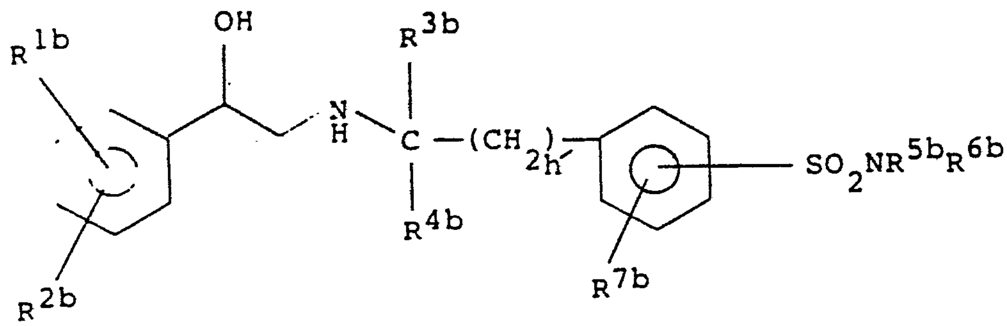

(XXVI)

or a salt thereof, wherein;

$R^{1b}$ is hydrogen, halogen or trifluoromethyl,

$R^{2b}$ is hydrogen or halogen,

$R^{3b}$ is hydrogen or methyl,

$R^{4b}$ is hydrogen or methyl,

$R^{5b}$ is hydrogen, $C_{1-10}$ straight or branched alkyl or optionally substituted benzyl,

$R^{6b}$ is hydrogen, $C_{1-10}$ straight or branched alkyl, or optionally substituted benzyl,

or $R^{5b}$ and $R^{6b}$ taken together with the attached nitrogen atom represent a heterocylic ring,

$R^{7b}$ is hydrogen or halogen, and

h' is 1.

The compounds of EP91749A are described as having anti-obesity and/or anti-hyperglycaemic activity coupled with low cardiac stimulant activity.

European Published Patent Application No. 0,063,004 discloses compounds of formula (XXVII):

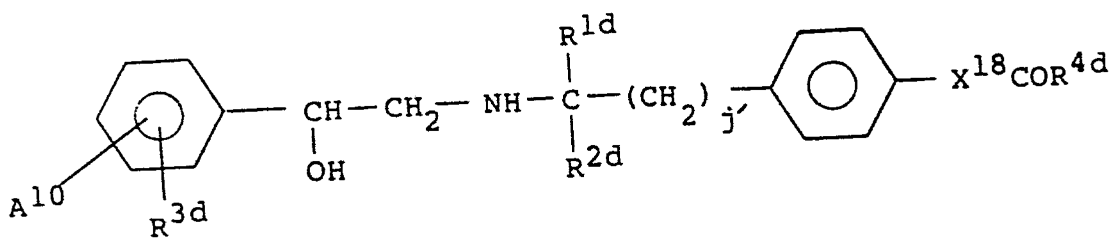

(XXVII)

and salts thereof;
wherein $R^{1d}$ is hydrogen or methyl:
$R^{2d}$ is hydrogen or methyl;
$R^{3d}$ is hydrogen, fluorine, chlorine, bromine, trifluoromethyl or $C_{1-6}$ alkyl;
j′ is 1 or 2;
$X^{18}$ is $C_{1-6}$ straight or branched alkylene;
$R^{4d}$ is $C_{1-6}$ alkoxy, hydroxy or amino optionally substituted with one or more lower alkyl groups; and
$A^{10}$ is hydrogen, fluorine, chlorine or bromine.

The compounds of EP63004A are described as having anti-obesity and/or anti-hyperglycaemic activity.

European Published Application No. 0,102,213 discloses compounds of formula (XXVIII):

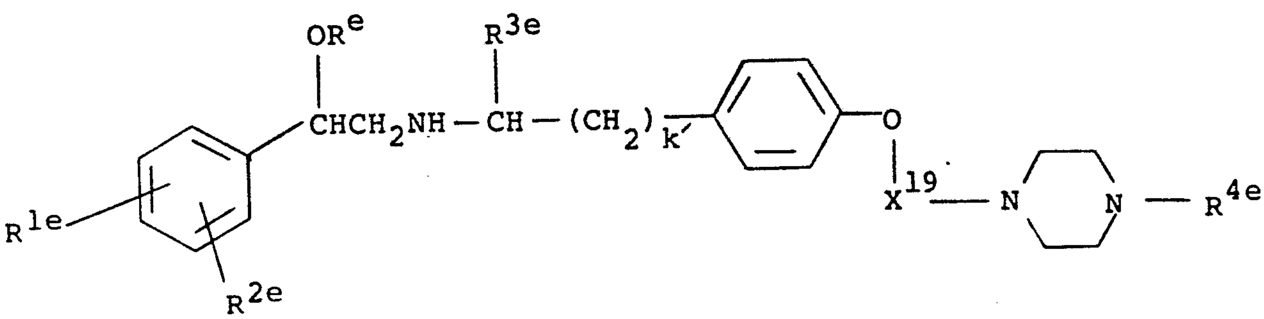

(XXVIII)

or a salt thereof,
wherein $R^e$ is hydrogen or $C_{1-6}$ alkyl,
$R^{1e}$ is hydrogen, or fluorine,
$R^{2e}$ is hydrogen, halogen or trifluoromethyl,
$R^{3e}$ is hydrogen or methyl,
$R^{4e}$ is hydrogen, $C_{1-6}$ alkyl, optionally substituted benzyl, or optionally substituted phenyl

$X^{19}$ is $C_{2-10}$ straight or branched alkylene having at least two carbon atoms interposed between the oxygen and nitrogen atoms

and k′ is 1 or 2.

The compounds of EP102213A are described as having anti-obesity and/or anti-hyperglycaemic activity coupled with low cardiac stimulant activity.

European Published Patent Application No. 0,196,849A discloses compounds of formula (XXIX).

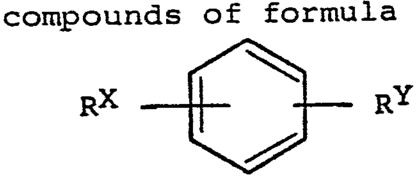

(XXIX)

or a pharmaceutically acceptable salt thereof, wherein $R^X$ is a group of formula (a):

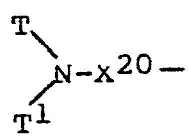

(a)

wherein T represents a group of formula (b):

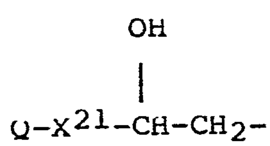

(b)

wherein Q is 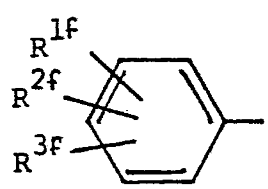 , and wherein

$R^{1f}$ is a hydrogen or halogen atom or a $-CF_3$, $OR^{4f}$, $NHR^{4f}$, $-NHCOR^{4f}$, $-NHCONH_2$, $NHSO_2R^{4f}$, $CH_2OR^{4f}$ or $-CH_2SO_2R^{4f}$ group;

$R^{2f}$ is a hydrogen or halogen atom or an $-OR^4$ group;

$R^{3f}$ is a hydrogen or halogen atom; and $R^{4f}$ is hydrogen, $C_{1-6}$ alkyl or benzyl;

$X^{20}$ is a group of formula $-CR^{5f}R^{6f}-CH_2-A^{11}-$

wherein $R^{5f}$ and $R^{6f}$ are independently hydrogen or $C_{1-6}$ alkyl;

$X^{21}$ is a bond or $-OCH_2-$; and

$A^{11}$ is a bond, $-CH_2-$, or O;

$T^1$ represents a hydrogen atom or a group of formula (b) as defined above in respect of T, such that $T^1$ and T may be the same or different; $R^Y$ represents a group of formula (a), as defined above in respect of $R^X$, such that $R^Y$ and $R^X$ may be the same or different.

The compounds of EP 196849A are described as having anti-obesity and/or anti-hyperglycaemic activity, they are also described as having useful growth promotion activity in animals.

European Patent Application No. 86309100 discloses compounds of the general formula (XXX):

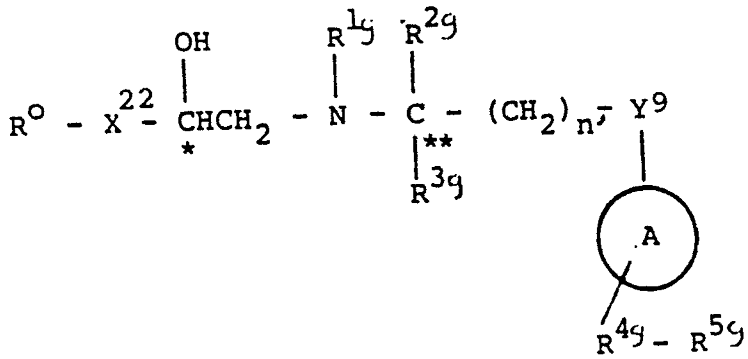

or a pharmaceutically acceptable ester thereof; or a pharmaceutically acceptable salt thereof,
wherein,

$R^o$ represents a substituted or unsubstituted aryl group or a substituted or unsubstituted benzofuranyl group,

$X^{22}$ represents a bond or $-O-CH_2-$ ,

$R^{1g}$ represents a hydrogen atom or a moiety

$$R^o-X^{22}-\underset{}{\overset{OH}{\overset{|}{C}}}HCH_2-$$ wherein $R^o$ and $X^{22}$ are as defined above;

***

$R^{2g}$ and $R^{3g}$ independently represent a hydrogen atom or an alkyl group,

n' represents an integer 1 or 2,

$Y^g$ represents a bond or a moiety $-CH_2-O-$,

moiety (A) represents an aryl group,

$R^{4g}$ represents a linking group, and

$R^{5g}$ represents a substituted or unsubstituted, monocyclic or fused ring heterocyclic group, having up to four heteroatoms in each ring selected from, oxygen, nitrogen and sulphur; provided that $R^5$ is not N-piperazinyl, N-piperadinyl, N-homopiperadinyl, N-pyrrolidinyl or N-morpholinyl.

The compounds of European Patent Application No. 86309100 are described as having anti-obesity and/or

anti-hyperglycaemic activity coupled with good selectivity from cardiac side effects.

It has now surprisingly been discovered that these compounds may be used to increase the high-density-lipoprotein (HDL) cholesterol concentration, to decrease the total cholesterol concentration and to decrease the triglyceride concentration in human blood serum and are therefore of use in the treatment and/or prophylaxis of atherosclerosis.

Accordingly, the present invention provides a method for increasing high-density lipoprotein (HDL) cholesterol concentration and/or decreasing total cholesterol concentration and/or decreasing triglyceride concentration in human blood serum, which method comprises the administration of an effective, non-toxic amount of a compound selected from the list consisting of compounds of the, hereinbefore defined, formulae: (I), (II), (III), (IV), (V), (VI), (VII), (VIII), (IX), (X), (XI), (XII), (XIII), (XIV), (XV), (XVI), (XVII), (XVIII), (XIX), (XX), (XXI), (XXII), (XXIII), (XXIV), (XXV), (XXVI), (XXVII), (XXVIII), (XXIX) and (XXX); or, where appropriate, a pharmaceutically acceptable salt, ester or amide thereof.

The present invention also encompasses the use of a compound selected from a list consisting of compounds of the, hereinbefore defined, formulae: (I), (II), (III), (IV), (V), (VI), (VII), (VIII), (IX), (X), (XI), (XII), (XIII), (XIV), (XV), (XVI), (XVII), (XVIII), (XIX), (XX), (XXI), (XXII), (XXIII), (XXIV), (XXV), (XXVI), (XXVII), (XXVIII), (XXIX) and (XXX); or where

appropriate a pharmaceutically acceptable salt, ester or amide thereof, for the manufacture of a medicament for increasing high-density-lipoprotein (HDL) cholesterol concentration and/or decreasing total cholesterol concentration and/or decreasing triglyceride concentration in human blood serum.

Suitably, the invention comprises the administration of a compound of formula (I) or a pharmaceutically acceptable salt, ester or amide thereof.

Suitably, the invention comprises the administration of a compound of formula (II) or a pharmaceutically acceptable salt, ester or amide thereof.

Suitably, the invention comprises the administration of a compound of formula (III) or a pharmaceutically acceptable salt, ester or amide thereof.

Suitably, the invention comprises the administration of a compound of formula (IV) or a pharmaceutically acceptable salt, ester or amide thereof.

Suitably, the invention comprises the administration of a compound of formula (V) or a pharmaceutically acceptable salt thereof.

Suitably, the invention comprises the administration of a compound of formula (VI) or a pharmaceutically acceptable salt thereof.

Suitably, the invention comprises the administration of a compound of formula (VII) or a pharmaceutically acceptable salt thereof.

Suitably, the invention comprises the administration of a compound of formula (VIII) or a pharmaceutically acceptable salt thereof.

Suitably, the invention comprises the administration of a compound of formula (IX) or a pharmaceutically acceptable salt or <u>in-vivo</u> hydrolysable acyl derivative thereof.

Suitably, the invention comprises the administration of a compound of formula (X) or a pharmaceutically acceptable salt thereof.

Suitably, the invention comprises the administration of a compound of formula (XI) or a pharmaceutically acceptable salt thereof.

Suitably, the invention comprises the administration of a compound of formula (XII) or a pharmaceutically acceptable salt, ester or amide thereof.

Suitably, the invention comprises the administration of a compound of formula (XIII) or a pharmaceutically acceptable salt, ester or amide thereof.

Suitably, the invention comprises the administration of a compound of formula (XIV) or a pharmaceutically acceptable salt, ester or amide thereof.

Suitably, the invention comprises the administration of a compound of formula (XV) or a pharmaceutically acceptable salt, ester or amide thereof.

Suitably, the invention comprises the administration of a compound of formula (XVI) or a pharmaceutically acceptable salt thereof.

Suitably, the invention comprises the administration of a compound of formula (XVII) or a pharmaceutically acceptable salt thereof.

Suitably, the invention comprises the administration of a compound of formula (XVIII) or a pharmaceutically acceptable salt, ester or amide thereof.

Suitably, the invention comprises the administration of a compound of formula (XIX) or a pharmaceutically acceptable salt, ester or amide thereof.

Suitably, the invention comprises the administration of a compound of formula (XX) or a pharmaceutically acceptable salt, ester or amide thereof.

Suitably, the invention comprises the administration of a compound of formula (XXI) or a pharmaceutically acceptable salt thereof.

Suitably, the invention comprises the administration of a compound of formula (XXII) or a pharmaceutically acceptable salt, ester or amide thereof.

Suitably, the invention comprises the administration of a compound of formula (XXIII) or a pharmaceutically acceptable salt, ester or amide thereof.

Suitably, the invention comprises the administration of a compound of formula (XXIV) or a pharmaceutically acceptable salt thereof.

Suitably, the invention comprises the administration of a compound of formula (XXV) or a pharmaceutically acceptable salt, ester or amide thereof.

Suitably, the invention comprises the administration of a compound of formula (XXVI) or a pharmaceutically acceptable salt, ester or amide thereof.

Suitably, the invention comprises the administration of a compound of formula (XXVII) or a pharmaceutically acceptable salt, ester or amide thereof.

Suitably, the invention comprises the administration of a compound of formula (XXVIII) or a pharmaceutically acceptable salt, ester or amide thereof.

Suitably, the invention comprises the administration of a compound of formula (XXIX) or a pharmaceutically acceptable salt, ester or amide thereof.

Suitably, the invention comprises the administration of a compound of formula (XXX) or a pharmaceutically acceptable salt, ester or amide thereof.

Preferably, the invention comprises the administration of a compound of formula (I) or (II) or a pharmaceutically acceptable salt, ester or amide thereof;

When the invention comprises the administration of a compound of formula (I) as herein before defined:

Apt values for $R^1$ include the hydrogen, fluorine and chlorine atoms and the hydroxymethyl, hydroxyl, methoxyl, acetamido, amino, methylsulphonylmethyl, methylsulphonamido, trifluoromethyl, ureido or p-methoxybenzylamino group.

Particularly suitable values for $R^2$ are the hydrogen and chlorine atoms.

Aptly $R^3$ is a hydrogen, hydroxyl or chlorine.

Particularly suitable groups $R^1R^2R^3C_6H_2$ include the phenyl; 3-ureido-4-hydroxyphenyl; 3-methane sulphonylamido-4-hydroxyphenyl; 3,5-dihydroxyphenyl; 3,4-dihydroxy-phenyl; 3-methylsulphonylmethyl -4-hydroxyphenyl; 3,5-dichloro-4-aminophenyl; 2-chlorophenyl; 2-methoxy-3,4-dihydroxyphenyl; 3-hydroxymethyl-4-hydroxyphenyl; 3-trifluoromethyl-phenyl; and 3-(p-methoxybenzyl)amino-4-hydroxyphenyl groups.

A preferred group $R^1R^2R^3C_6H_2$ is the phenyl group.

Another preferred group $R^1R^2R^3C_6H_2$ is the 3,5-dichloro-4-aminophenyl group. A further preferred group $R^1R^2R^3C_6H_2$ is the 3-hydroxy-methyl -4-hydroxyphenyl group. A further preferred group $R^1R^2R^3C_6H_2$ is the 3-trifluoromethylphenyl group. Apt groups of the formula $R^4$ include those of the sub-formulae (a) - (e):

| | |
|---|---|
| $-CO_2H$ | (a) |
| $-CO_2^-$ $1/q$ $A^{q+}$ | (b) |
| $-CO_2R_8$ | (c) |
| $-CO.NH_2$ | (d) |
| $-CO.NR_9R_{10}$ | (e) |

wherein $A^{q+}$ is an ion wherein q is aptly 1 or 2; $R^8$ is a group such that $CO_2R^8$ is an ester group; and $R^9$ is a lower alkyl group and $R_{10}$ is a hydrogen atom or a lower alkyl group or is joined to $R^9$ to form a saturated 5, 6 or 7 membered ring.

When used herein the term ''lower'' means that the group contains not more than 6 carbon atoms, preferably not more than 4.
Particularly apt values for $R^4$ include those of the sub-formulae (a), (b) or (c).

An especially favoured value for $R^4$ is that of the sub-formula (c). In such compounds it is suitable that the moiety $R^8$ is such that the ester group is hydrolysed in-vivo to yield the corresponding compound wherein $R^4$ is a group of the sub-formula (a) or a salt thereof.

Particularly suitable values for $R^8$ include lower alkyl groups, lower alkyl groups substituted by a hydroxyl group not on the α-carbon atom and groups of the sub-formulae (f) or (g):

$$-CHR_{11}-O-CO-R_{12} \qquad (f)$$

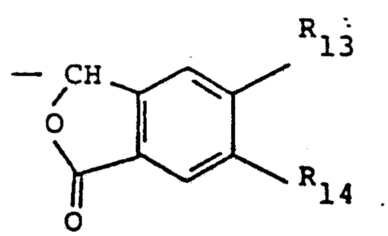

(g)

wherein $R^{11}$ is a hydrogen atom or a methyl group; $R^{12}$ is a lower alkyl or phenyl group; $R^{13}$ is a hydrogen atom or a methyl or methoxyl group; and $R^{14}$ is a hydrogen atom or a methyl or methoxyl group.

Certain particularly suitable values for $R^8$ include the methyl, ethyl, propyl and butyl groups, for example the methyl group and the isopropyl group.

Preferably $R^8$ is a methyl group.

The point of attachment of the group $R^4$ is aptly meta- or para- to the point of attachment of the phenyl group to the rest of the molecule.

Suitably $R^5$ is hydrogen or methoxyl.

Preferably $R^5$ is hydrogen.

A favourable value for $R^6$ is a hydrogen atom. A further favourable value for $R^6$ is the methyl group. A favourable value for $R^7$ is the hydrogen atom. A further favourable value for $R^7$ is the methyl group. Most favourably $C(R^6)R^7$ is a $CH_2$, $CH(CH_3)$, or $C(CH_3)_2$ group, preferably $CH(CH_3)$.

Suitably, when the invention comprises the administration of a compound of formula (II), as hereinbefore defined, or a pharmaceutically acceptable salt, ester or amide thereof the $O-Z-CO_2H$ moiety or the said salt, ester or amide thereof, is attached para- to the $-X^1-$ moiety.

Suitable values for $R^{1A}$ include the hydrogen, fluorine, chlorine and bromine atoms and the trifluoromethyl, hydroxymethyl, hydroxyl and amino groups.

Suitably $X^1$ in the compounds of the formula (II) is a bond.

Preferred groups $Y^1$ are of the formula $-(CH_2)_{n'}-$ where n′ is an integer from 1 to 5, particularly 1 or 2.

Particularly suitable values for each of $R^{2A}$ and $R^{3A}$ are the hydrogen and chlorine atoms.

Particularly suitable groups $R^{1A}R^{2A}R^{3A}C_6H_2$ include the phenyl; 2-fluorophenyl; 3-trifluoromethylphenyl; 3-chlorophenyl, 3,5-dichloro-4-aminophenyl; 2-chlorophenyl; 3-hydroxymethyl-4-hydroxyphenyl; 3-chlorophenyl; and 3,5-dichloro-4-amino groups, preferably 3-chlorophenyl.

In accordance with conventional usage, the terms ''alkenylene'' and ''alkynylene'' do not extend to systems containing an oxygen atom attached to the carbon of a carbon-carbon double bond.

The group Z may be branched, for example to carry one or two methyl groups, but it is more suitably unbranched. Aptly the group Z contains 1 to 6 carbon atoms and more suitably 1 to 4 carbon atoms.

Groups Z include $CH_2$, $CH_2CH_2$, $CH_2CH_2CH_2$, $CH_2CH_2CH_2CH_2$, $CH_2CH=CH$.

A particularly favoured group Z is the $CH_2$ group.

Favourably, the invention comprises the administration of an ester of the compound of formula (II) including those esters that are hydrolysed in-vivo to yield the corresponding compound of formula (II) per se or its salt.

Particularly suitable in-vivo hydrolysable ester forming groups include those of formula (c) as defined above, such as lower alkyl groups, lower alkyl groups substituted by a hydroxyl group not on the α-carbon atom and groups of the sub-formulae (f) and (g) as hereinbefore defined.

In a preferred aspect the method of the invention comprises the administration of a preferred sub-group of the compounds of formula (I), being compounds of formula (IIIA):

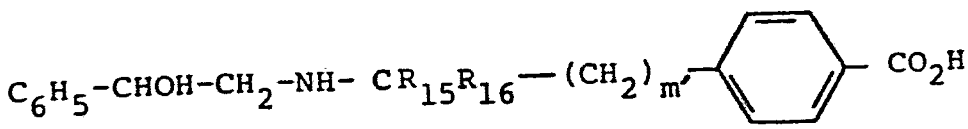

(IIIA)

or a pharmaceutically acceptable salt, ester or amide thereof, wherein $R^{15}$ is a hydrogen atom or a methyl group; $R^{16}$ is a hydrogen atom or a methyl group; and m′ is 1,2 or 3.

Most suitably, $R^{15}$ is a hydrogen atom.

Most suitably $R^{16}$ is a methyl group.

Preferably m′ is 1.

Esters of the compound of the formula (IIIA) include those of the sub-formulae (c), (f) and (g) as defined in relation to formula (I).

A particularly preferred ester of the compound of formula (IIIA) is the methyl ester.

In a second preferred aspect the invention comprises the administration of another preferred sub-group of the compounds of formula (I), being compounds of formula (IVA):

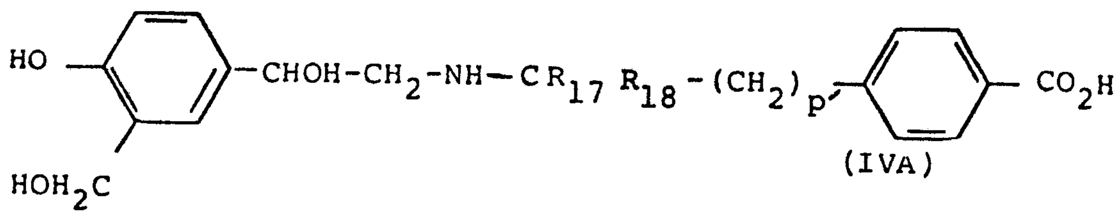

(IVA)

or a pharmaceutically acceptable salt, ester or amide thereof wherein $R^{17}$ is a hydrogen atom or a methyl group; $R^{18}$ is a hydrogen atom or a methyl group; and $p'$ is 1, 2 or 3.

Most suitably $R^{17}$ is a hydrogen atom. Most suitably $R^{18}$ is a methyl group.
Preferably $p'$ is 1.

Esters of the compounds of the formula (IVA) include those of the sub-formulae (c), (f) and (g) as defined in relation to formula (I).

A particularly preferred ester of the compound of formula (IVA) is the methyl ester.

In a further preferred aspect, the invention comprises the administration of a preferred sub-group of the compounds of formula (II), being compounds of formula (VA):

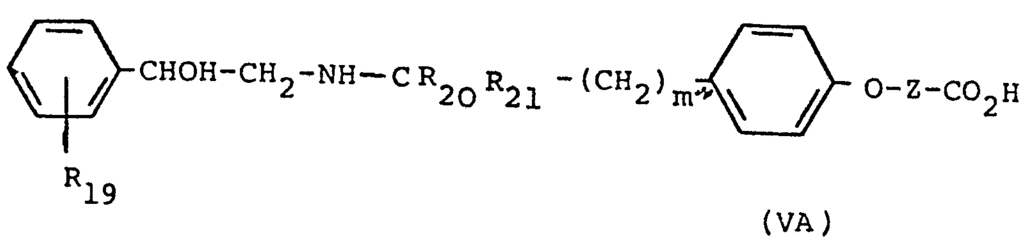

(VA)

or a pharmaceutically acceptable salt, ester or amide thereof, wherein $R^{19}$ is a hydrogen, fluorine or chlorine atom or a trifluoromethyl group, Z is as defined in relation to formula (II), $R^{20}$ is a hydrogen atom or a methyl group; $R^{21}$ is a hydrogen atom or a methyl group; and m'' is 1, 2 or 3.
Preferably Z is a $CH_2$ group.
Suitably, $R^{19}$ is attached meta to the -CHOH- moiety.
Preferably $R^{19}$ is a chlorine atom. A preferred group $R^{19}C_6H_4$- is the 3-chlorophenyl group.
Preferably $R^{20}$ is hydrogen.
Preferably $R^{21}$ is methyl.
Preferably m'' is 1.
Esters of the compounds of formula (VA) include those of sub-formulae (c), (f) and (g) as defined in relation to formula (I). A particularly preferred ester of the compound of formula (VA) is a methyl ester.

Certain compounds of the invention (for example the compounds formula (I), wherein $R^4$ is other than a carboxylic acid salt, and the compounds of formula (II) which are esterified) may be provided as acid addition salts. Such salts may be of an organic or inorganic acid but are normally salts with a pharmaceutically acceptable acid. Suitable acid addition salts include those formed with acids such as hydrochloric, hydrobromic, orthophosphoric, sulphuric, methanesulphonic, toluenesulphonic, acetic, propionic, lactic, citric, fumaric, malic, succinic, salicylic, acetylsalicylic or the like acids.

A preferred acid addition salt of the compound of formula (I) is the fumarate salt, preferably the hemi-fumarate salt.

In addition to the above mentioned compounds, further suitable compounds of formula (I) or where appropriate,

salts, esters or amides thereof, include those disclosed in EP 0006735. Preferred compounds of formula (I), or where appropriate salts, esters or amides thereof, include those disclosed in EP 0006735; it will be understood that the above mentioned suitable and preferred compounds of formula (I) include the suitable and preferred stereochemical isomers of the compounds of formula (I) disclosed in EP 0006735.

In addition to the above mentioned compounds, further suitable compounds of formula (II) or where appropriate, salts, esters or amides thereof, include those disclosed in EP 0023385. Preferred compounds of formula (II), or where appropriate salts, esters or amides thereof, include those disclosed in EP 0023385; it will be understood that the above mentioned suitable and preferred compounds of formula (II) include the suitable and preferred stereochemical isomers of the compounds of formula (II) disclosed in EP 0023385.

Suitable compounds of formula (III) or where appropriate, salts, esters or amides thereof, include those disclosed in EP0028105. Preferred compounds of formula (III), or where appropriate salts, esters or amides thereof, include those disclosed in EP0028105; it will be understood that the above mentioned suitable and preferred compounds of formula (III) include the suitable and preferred stereochemical isomers of the compounds of formula (III) disclosed in EP0028105.

Suitable compounds of formula (IV) or where appropriate, salts, esters or amides thereof, include those disclosed in EP0040915. Preferred compounds of formula (IV), or where appropriate salts, esters or amides thereof, include those disclosed in EP0040915; it will be understood that the above mentioned suitable

and preferred compounds of formula (IV) include the suitable and preferred stereochemical isomers of the compounds of formula (IV) disclosed in EP0040915.

Suitable compounds of formula (V) or where appropriate, salts, esters or amides thereof, include those disclosed in EP0040000. Preferred compounds of formula (V), or where appropriate salts, esters or amides thereof, include those disclosed in EP0040000; it will be understood that the above mentioned suitable and preferred compounds of formula (V) include the suitable and preferred stereochemical isomers of the compounds of formula (V) disclosed in EP0040000.

Suitable compounds of formula (VI) or where appropriate, salts, esters or amides thereof, include those disclosed in EP0052963A. Preferred compounds of formula (VI), or where appropriate salts, esters or amides thereof, include those disclosed in EP0052963A; it will be understood that the above mentioned suitable and preferred compounds of formula (VI) include the suitable and preferred stereochemical isomers of the compounds of formula (VI) disclosed in EP0052963A.

Suitable compounds of formula (VII) or where appropriate, salts, esters or amides thereof, include those disclosed in EP0066351.
Preferred compounds of formula (VII), or where appropriate salts, esters or amides thereof, include those disclosed in EP0066351; it will be understood that the above mentioned suitable and preferred compounds of formula (VII) include the suitable and preferred stereochemical isomers of the compounds of formula (VII) disclosed in EP0066351.

Suitable compounds of formula (VIII) or where appropriate, salts, esters or amides thereof, include those disclosed in EP0061907. Preferred compounds of formula (VIII), or where appropriate salts, esters or amides thereof, include those disclosed in EP0061907; it will be understood that the above mentioned suitable and preferred compounds of formula (VIII) include the suitable and preferred stereochemical isomers of the compounds of formula (VIII) disclosed in EP0061907.

Suitable compounds of formula (IX) or where appropriate, salts, esters or amides thereof, include those disclosed in EP0070133A. Preferred compounds of formula (IX), or where appropriate salts, esters or amides thereof, include those disclosed in EP0070133A;it will be understood that the above mentioned suitable and preferred compounds of formula (IX) include the suitable and preferred stereochemical isomers of the compounds of formula (IX) disclosed in EP0070133A.

Suitable compounds of formula (X) or where appropriate, salts, esters or amides thereof, include those disclosed in EP0070134 and GB1574208. Preferred compounds of formula (X), or where appropriate salts, esters or amides thereof, include those disclosed in EP0070134 and GB1574208; it will be understood that the above mentioned suitable and preferred compounds of formula (X) include the suitable and preferred stereochemical isomers of the compounds of formula (X) disclosed in EP0070134 and GB1574208.

Suitable compounds of formula (XI) or where appropriate, salts, esters or amides thereof, include those disclosed in EP0095827. Preferred compounds of

formula (XI), or where appropriate salts, esters or amides thereof, include those disclosed in EP0095827; it will be understood that the above mentioned suitable and preferred compounds of formula (XI) include the suitable and preferred stereochemical isomers of the compounds of formula (XI) disclosed in EP0095827.

Suitable compounds of formula (XII) or where appropriate, salts, esters or amides thereof, include those disclosed in EP0099707. Preferred compounds of formula (XII), or where appropriate salts, esters or amides thereof, include those disclosed in EP0099707; it will be understood that the above mentioned suitable and preferred compounds of formula (XII) include the suitable and preferred stereochemical isomers of the compounds of formula (XII) disclosed in EP0099707.

Suitable compounds of formula (XIII) or where appropriate, salts, esters or amides thereof, include those disclosed in EP0139921A. Preferred compounds of formula (XIII), or where appropriate salts, esters or amides thereof, include those disclosed in EP0139921A;it will be understood that the above mentioned suitable and preferred compounds of formula (XIII) include the suitable and preferred stereochemical isomers of the compounds of formula (XIII) disclosed in EP0139921A.

Suitable compounds of formula (XIV) or where appropriate, salts, esters or amides thereof, include those disclosed in EP0142102A. Preferred compounds of formula (XIV), or where appropriate salts, esters or amides thereof, include those disclosed in EP0142102A;it will be understood that the above mentioned suitable and preferred compounds of formula (XIV) include the suitable and preferred stereochemical

isomers of the compounds of formula (XIV) disclosed in EP0142102A.

Suitable compounds of formula (XV) or where appropriate, salts, esters or amides thereof, include those disclosed in EP0164700A. Preferred compounds of formula (XV), or where appropriate salts, esters or amides thereof, include those disclosed in EP0164700A; it will be understood that the above mentioned suitable and preferred compounds of formula (XV) include the suitable and preferred stereochemical isomers of the compounds of formula (XV) disclosed in EP0164700A.

Suitable compounds of formula (XVI) or where appropriate, salts, esters or amides thereof, include those disclosed in EP0170121A. Preferred compounds of formula (XVI), or where appropriate salts, esters or amides thereof, include those disclosed in EP0170121A; it will be understood that the above mentioned suitable and preferred compounds of formula (XVI) include the suitable and preferred stereochemical isomers of the compounds of formula (XVI) disclosed in EP0170121A.

Suitable compounds of formula (XVII) or where appropriate, salts, esters or amides thereof, include those disclosed in EP0170702A Preferred compounds of formula (XVII), or where appropriate salts, esters or amides thereof, include those disclosed in EP0170702A; it will be understood that the above mentioned suitable and preferred compounds of formula (XVII) include the suitable and preferred stereochemical isomers of the compounds of formula (XVII) disclosed in EP0170702A.

Suitable compounds of formula (XVIII) or where appropriate, salts, esters or amides thereof, include those disclosed in EP0029320. Preferred compounds of

formula (XVIII), or where appropriate salts, esters or amides thereof, include those disclosed in EP0029320; it will be understood that the above mentioned suitable and preferred compounds of formula (XVIII) include the suitable and preferred stereochemical isomers of the compounds of formula (XVIII) disclosed in EP0029320.

Suitable compounds of formula (XIX) or where appropriate, salts, esters or amides thereof, include those disclosed in EP0051917A. Preferred compounds of formula (XIX), or where appropriate salts, esters or amides thereof, include those disclosed in EP0051917A; it will be understood that the above mentioned suitable and preferred compounds of formula (XIX) include the suitable and preferred stereochemical isomers of the compounds of formula (XIX) disclosed in EP0051917A.

Suitable compounds of formula (XX) or where appropriate, salts, esters or amides thereof, include those disclosed in EP0140359A. Preferred compounds of formula (XX), or where appropriate salts, esters or amides thereof, include those disclosed in EP0140359A; it will be understood that the above mentioned suitable and preferred compounds of formula (XX) include the suitable and preferred stereochemical isomers of the compounds of formula (XX) disclosed in EP0140359A.

Suitable compounds of formula (XXI) or where appropriate, salts, esters or amides thereof, include those disclosed in EP0171519A. Preferred compounds of formula (XXI), or where appropriate salts, esters or amides thereof, include those disclosed in EP0171519A; it will be understood that the above mentioned suitable and preferred compounds of formula (XXI) include the suitable and preferred stereochemical isomers of the compounds of formula (XXI) disclosed in EP0171519A.

Suitable compounds of formula (XXII) or where appropriate, salts, esters or amides thereof, include those disclosed in EP170135A. Preferred compounds of formula (XXII), or where appropriate salts, esters or amides thereof, include those disclosed in EP170135A; it will be understood that the above mentioned suitable and preferred compounds of formula (XXII) include the suitable and preferred stereochemical isomers of the compounds of formula (XXII) disclosed in EP170135A.

Suitable compounds of formula (XXIII) or where appropriate, salts, esters or amides thereof, include those disclosed in EP0021636. Preferred compounds of formula (XXIII), or where appropriate salts, esters or amides thereof, include those disclosed in EP0021636; it will be understood that the above mentioned suitable and preferred compounds of formula (XXIII) include the suitable and preferred stereochemical isomers of the compounds of formula (XXIII) disclosed in EP0021636.

Suitable compounds of formula (XXIV) or where appropriate, salts, esters or amides thereof, include those disclosed in EP0025331. Preferred compounds of formula (XXIV), or where appropriate salts, esters or amides thereof, include those disclosed in EP0025331 it will be understood that the above mentioned suitable and preferred compounds of formula (XXIV) include the suitable and preferred stereochemical isomers of the compounds of formula (XXIV) disclosed in EP0025331.

Suitable compounds of formula (XXV) or where appropriate, salts, esters or amides thereof, include those disclosed in EP0089154A Preferred compounds of formula (XXV), or where appropriate salts, esters or amides thereof, include those disclosed in EP0089154A; it will be understood that the above mentioned suitable

and preferred compounds of formula (XXV) include the suitable and preferred stereochemical isomers of the compounds of formula (XXV) disclosed in EP0089154A.

Suitable compounds of formula (XXVI) or where appropriate, salts, esters or amides thereof, include those disclosed in EP0091749A. Preferred compounds of formula (XXVI), or where appropriate salts, esters or amides thereof, include those disclosed in EP0091749A; it will be understood that the above mentioned suitable and preferred compounds of formula (XXVI) include the suitable and preferred stereochemical isomers of the compounds of formula (XXVI) disclosed in EP0091749A.

Suitable compounds of formula (XXVII) or where appropriate, salts, esters or amides thereof, include those disclosed in EP0063004A. Preferred compounds of formula (XXVII), or where appropriate salts, esters or amides thereof, include those disclosed in EP0063004A; it will be understood that the above mentioned suitable and preferred compounds of formula (XXVII) include the suitable and preferred stereochemical isomers of the compounds of formula (XXVII) disclosed in EP0063004A.

Suitable compounds of formula (XXVIII) or where appropriate, salts, esters or amides thereof, include those disclosed in EP0102213. Preferred compounds of formula (XXVIII), or where appropriate salts, esters or amides thereof, include those disclosed in EP0102213; it will be understood that the above mentioned suitable and preferred compounds of formula (XXVIII) include the suitable and preferred stereochemical isomers of the compounds of formula (XXVIII) disclosed in EP0102213.

Suitable compounds of formula (XXIX) or where appropriate, salts, esters or amides thereof, include

those disclosed in EP0196849A. Preferred compounds of formula (XXIX), or where appropriate salts, esters or amides thereof, include those disclosed in EP0196849A; it will be understood that the above mentioned suitable and preferred compounds of formula (XXIX) include the suitable and preferred stereochemical isomers of the compounds of formula (XXIX) disclosed in EP0196849A.

Suitable compounds of formula (XXX) or where appropriate, salts, esters or amides thereof, include those disclosed in European Patent Application No. 86309100. Preferred compounds of formula (XXX), or where appropriate salts, esters or amides thereof, include those disclosed in European Patent Application No. 86309100; it will be understood that the above mentioned suitable and preferred compounds of formula (XXX) include the suitable and preferred stereochemical isomers of the compounds of formula (XXX) disclosed in European Patent Application No. 86309100.

The carbon atoms marked with a single asterisk ''*'' in compounds (I) and (II) are asymmetric carbon atoms.

In the compounds of formula (I) and (II) the carbon atoms marked with a double asterisk ''**'' are asymmetric carbon atoms, when $R^6 = R^7$ and $R^{4A} = R^{5A}$ respectivly.

It will be clear from the above that the compounds of formula (I) or (II) may exist in at least two and often four stereoisomeric forms. The present invention encompasses the administration of all stereoisomers of the compounds of formula (I) whether free from other stereoisomers or admixed with other stereoisomers in any proportion and hence includes, for instance, racemic mixtures of enantiomers.

Suitably, in the compounds of formula (I), the ''*'' asymmetric carbon has the R-configuration.

Suitably, in the compounds of formula (II), the ''*'' asymmetric carbon has the R-configuration.

Suitably, in the compounds of formula (I), when $R^6$ = $R^7$, the ''**'' asymmetric carbon has the R-configuration.

Suitably, in the compounds of formula (II), when $R^{4A}$ = $R^{5A}$, the ''**'' asymmetric carbon has the R-configuration.

Preferably, in the compounds of formula (I), when $R_6$ = $R^7$, a preferred enantiomer is that wherein:
the ''*'' asymmetric carbon = R- configuration; and
the ''*'' asymmetric carbon = R- configuration;
that is the RR enantiomer.

It will therefore be understood that a preferred diastereoisomer of the compounds of formula (I) is the RR:SS diastereoisomer.

Preferably, in the compounds of formula (II), when $R^{4A}$ = $R^{5A}$, a preferred enantiomer is that wherein:
the ''*'' asymmetric carbon = R-configuration; and
the ''**'' asymmetric carbon = R-configuration,
that is the RR enantiomer.

It will therefore be understood that a preferred diastereoisomer of the compounds of formula (II) is the RR:SS diastereoisomer.

Compounds of formula (I) or (II) having a single asymmetric carbon atom may, if desired, be separated

into individual enantiomers by conventional means, for example by the use of an optically active acid as a resolving agent. Those compounds of formula (I) or (II) having two asymmetric carbon atoms may be separated into diastereoisomic pairs of enantiomers by, for example fractional crystallisation from a suitable solvent such as methanol or ethyl acetate or a mixture thereof. The pair of enantiomers thus obtained may be separated into individual stereoisomers by conventional means such as by the use of an optically active acid as a resolving agent.

Suitable optically active acids which may be used as resolving agents are described in ''Topics in Stereochemistry'' Vol 6, Wiley Interscience, 1971, Allinger, N.L. and Eliel, W.L. Eds.

Alternatively any enantiomer of a compound of formula (I) or (II) may be obtained by stereospecific synthesis using an optically pure starting material of known configuration.

Similarly, the present invention extends to administration of the individual steroisomeric forms of the compounds of formulae (III), (IV), (V), (VI), (VII), (VIII), (IX), (X), (XI),(XII), (XIII), (XIV), (XV), (XVI), (XVII), XVIII), (XIX), (XX), (XXI), (XXII), (XXIII), (XXIV), (XXV), (XXVI), (XXVII), (XXVIII), (XXIX) and (XXX). The preferred sterochemical isomers of each of these compounds and the methods of obtaining them are as indicated above.

Preferably, the invention comprises the administration of a compound selected from the list consisting of:

N-[2-(4-carbomethoxyphenyl)-1-methylethyl]-2-hydroxy-2-(4-hydroxy-3-hydroxymethylphenyl)ethanamine;

N-[2-(4-carboxyphenyl)-1-methylethyl]-2-hydroxy-2-(4-hydroxy-3-hydroxymethylphenyl)ethanamine;

N-[2-(4-carboxyphenyl)-1-methylethyl]-2-hydroxy-2-phenylethanamine;

N-[2-(4-carbomethoxyphenyl)-1-methylethyl]-2-hydroxy-2-phenylethanamine;

N-[2-(4-carbomethoxyphenyl)-1-methylethyl]-2-hydroxy-2-(4-amino-3,5-dichlorophenyl)ethanamine;

N-[2-(4-carboxyphenyl)-1-methylethyl]-2-hydroxy-2-(4-amino-3,5-dichlorophenyl)ethanamine;

N-[2-(4-carbomethoxymethoxyphenyl)-1-methylethyl]-2-hydroxy-2-(4-amino-3,5-dichlorophenyl) ethanamine;

N-[2-(4-carboxymethoxyphenyl)-1-methylethyl]-2-hydroxy-2-(4-amino-3,5-dichlorophenyl) ethanamine;

N-[2-(4-carbomethoxymethoxyphenyl)-1-methylethyl]-2-hydroxy-2-(3-chlorophenyl) ethanamine; and

N-[2-(4-carboxymethoxyphenyl)-1-methylethyl]-2-hydroxy-2-(3-chlorophenyl) ethanamine; or a pharmaceutically acceptable acid addition salt thereof.

The compounds of formula (1), (IIIA) and (IVA) may suitably be prepared by conventional methods including those disclosed in EP 0,006,735.

The compounds of formula (IIA) and (VA) may suitably be prepared by conventional methods including those disclosed in EP 0,023,385.

Suitably, the compounds of formula (III), or where appropiate, salts, esters and amides thereof, are prepared by methods conventional in the art, for example those disclosed in EP0028105.

Suitably, the compounds of formula (IV), or where appropiate, salts, esters and amides thereof, are prepared by methods conventional in the art, for example those disclosed in EP0040915.

Suitably, the compounds of formula (V), or where appropiate, salts, esters and amides thereof, are prepared by methods conventional in the art, for example those disclosed in EP0040000.

Suitably, the compounds of formula (VI) or where appropiate, salts, esters and amides thereof, are prepared by methods conventional in the art, for example those disclosed in EP0052963A.

Suitably, the compounds of formula (VII) or where appropiate, salts, esters and amides thereof, are prepared by methods conventional in the art, for example those disclosed in EP0066351.

Suitably, the compounds of formula (VIII) or where appropiate, salts, esters and amides thereof, are prepared by methods conventional in the art, for example those disclosed in EP0061907.

Suitably, the compounds of formula (IX), or where appropiate, salts, esters and amides thereof, are

prepared by methods conventional in the art, for example those disclosed in EP0070133A.

Suitably, the compounds of formula (X), or where appropiate, salts, esters and amides thereof, are prepared by methods conventional in the art, for example those disclosed in EP0070134 and GB1574208.

Suitably, the compounds of formula (XI), or where appropiate, salts, esters and amides thereof, are prepared by methods conventional in the art, for example those disclosed in EP0095827.

Suitably, the compounds of formula (XII), or where appropiate, salts, esters and amides thereof, are prepared by methods conventional in the art, for example those disclosed in EP0099707.

Suitably, the compounds of formula (XIII), or where appropiate, salts, esters and amides thereof, are prepared by methods conventional in the art, for example those disclosed in EP0139921.

Suitably, the compounds of formula (XIV), or where appropiate, salts, esters and amides thereof, are prepared by methods conventional in the art, for example those disclosed in EP0142102A.

Suitably, the compounds of formula (XV), or where appropiate, salts, esters and amides thereof, are prepared by methods conventional in the art, for example those disclosed in EP0164700A.

Suitably, the compounds of formula (XVI) or where appropiate, salts, esters and amides thereof, are

prepared by methods conventional in the art, for example those disclosed in EP017121A.

Suitably, the compounds of formula (XVII), or where appropiate, salts, esters and amides thereof, are prepared by methods conventional in the art, for example those disclosed in EP0170702A.

Suitably, the compounds of formula (XVIII), or where appropiate, salts, esters and amides thereof, are prepared by methods conventional in the art, for example those disclosed in EP0029320.

Suitably, the compounds of formula (XIX) or where appropiate, salts, esters and amides thereof, are prepared by methods conventional in the art, for example those disclosed in EP0051917A.

Suitably, the compounds of formula (XX), or where appropiate, salts, esters and amides thereof, are prepared by methods conventional in the art, for example those disclosed in EP0140359A.

Suitably, the compounds of formula (XXI), or where appropiate, salts, esters and amides thereof, are prepared by methods conventional in the art, for example those disclosed in EP0170135A.

Suitably, the compounds of formula (XXII), or where appropiate, salts, esters and amides thereof, are prepared by methods conventional in the art, for example those disclosed in EP0170135A

Suitably, the compounds of formula (XXIII), or where appropriate, salts, esters and amides thereof, are

prepared by methods conventional in the art, for example those disclosed in EP0021636.

Suitably, the compounds of formula (XXIV) or where appropriate, salts, esters and amides thereof, are prepared by methods conventional in the art, for example those disclosed in EP0025331.

Suitably, the compounds of formula (XXV), or where appropriate, salts, esters and amides thereof, are prepared by methods conventional in the art, for example those disclosed in EP0089154A.

Suitably, the compounds of formula (XXVI), or where appropriate, salts, esters and amides thereof, are prepared by methods conventional in the art, for example those disclosed in EP0091749A.

Suitably, the compounds of formula (XXVII), or where appropriate, salts, esters and amides thereof, are prepared by methods conventional in the art, for example those disclosed in EP0063004A.

Suitably, the compounds of formula (XXVII), or where appropriate, salts, esters and amides thereof, are prepared by methods conventional in the art, for example those disclosed in EP0102213.

Suitably, the compounds of formula (XXIX), or where appropriate, salts, esters and amides thereof, are prepared by methods conventional in the art, for example those disclosed in EP0196849A.

Suitably, the compounds of formula (XXX), or where appropriate, salts, esters and amides thereof, are prepared by methods conventional in the art, for

example those disclosed in European Patent Application No. 86309100.

A compound of formulae (I), (II), (III), (IV), (V), (VI), (VII), (VIII), (IX), (X), (XI), (XII), (XIII), (XIV), (XV), (XVI), (XVII), (XVIII), (XIX), (XX), (XXI), (XXII), (XXIII), (XXIV), (XXV), (XXVI), (XXVII), (XXVIII), (XXIX) or (XXX); or where appropriate, a pharmaceutically acceptable salt, ester or amide thereof ('the drug'), will normally be administered <u>per se</u> or, preferably, as a pharmaceutical composition also comprising a pharmaceutically acceptable carrier.

Accordingly, the present invention also provides a pharmaceutical composition for increasing high-density-lipoprotein (HDL) cholesterol concentration and/or decreasing total cholesterol concentration and/or decreasing triglyceride concentration in human blood serum which composition comprises a compound selected from the list consisting of compounds of the, hereinbefore defined, formulae: (I), (II), (III), (IV), (V), (VI), (VII), (VIII), (IX), (X), (XI), (XII), (XIII), (XIV), (XV), (XVI), (XVII), (XVIII), (XIX), (XX), (XXI), (XXII), (XXIII), (XXIV), (XXV), (XXVI), (XXVII), (XXVIII), (XXIX) and (XXX); or a pharmaceutically acceptable salt, ester or amide thereof with a pharmaceutically acceptable carrier therefor.

As used herein the term ''pharmaceutically acceptable'' embraces compounds, compositions and ingredients for both human and veterinary use: for example the term ''pharmaceutically acceptable salt'' embraces a veterinarily acceptable salt.

The composition may, if desired, be in the form of a pack accompanied by written or printed instructions for use.

Usually in the method of the invention the drug will be adapted for oral administration, although administration by other routes, such as by injection, are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventional adjuvant.

Typical carriers include, for example, microcrystalline cellulose, starch, sodium starch glycollate, polyvinylpyrrolidone, polyvinylpolypyrrolidone, magnesium stearate, sodium lauryl sulphate or sucrose.

Most suitably the composition will be formulated in unit dose form. Such unit dose will normally contain an amount of the active ingredient in the range of from 0.1 to 1000 mg, more usually 0.1 to 500 mg, and more especially 0.1 to 250 mg.

Conveniently, the drug may be administered as a pharmaceutical composition hereinbefore defined, and this forms a particular aspect of the present invention.

In the method of the present invention the drug may be taken in doses, such as those described above, one to six times a day in a manner such that the total daily dose for a 70 kg adult will generally be in the range of from 0.1 to 6000 mg, and more usually about 1 to 1500 mg.

The following Examples illustrate the invention but do not limit it in any way.

Preparations:

P1:- N-(2-(4-carbomethoxphenyl)-1-metnyletnyl)-2-hydroxy-2-(4-hydroxy-3-hydroxymethylphenyl) ethanamine may be prepared by the method described in Example 1 of EP23385A.

P2:- N-(2-(4-carbomethoxymethoxyphenyl)-1-methyletnyl)--2-hydroxy-2-(3-chlorophenyl)ethanamine nydrobromide may be prepared as the racemic mixture of the RR and SS stereoisomers using the method described in Example 6 of EP23385A.

P3:- N-[2-(4-carbomethoxyphenyl)-1-methylethyl]-2-hydroxy-2-phenylethanamine hemifumarate was prepared as the racemic mixture of the RR and SS stereoisomers using methods described in EP6735, Example 21.

Example 1:

Compound P1 provided the following results with normal mice:

Normal female CFLP mice were dosed daily at 11.00h for 28 days. One group was also dosed at 17.00h daily. On the day after dosing terminated they were killed at 14.00h. Results are means of 8 values ± S.E.

| Dose level (mg/kg body wt.) (p.o.) | Serum triglyceride (mg/100 ml) | Serum cholesterol (mg/100 ml) |
|---|---|---|
| 0 | 40.0±3.2 | 67.6±2.4 |
| 2.5 | 21.5±1.1* | 67.0±3.2 |
| 2.5 twice daily | 16.4±0.8* | 74.4±1.7 |
| 5 | 16.3±1.1* | 69.1±2.4 |
| 10 | 14.5±0.7* | 68.4±3.3 |
| 15 | 14.3±0.8* | 71.0±2.4 |

* Significantly different from value for untreated mice, $P < 0.001$.

Example 2:

Compound P1 provided the following results with Goldthioglucose-induced obese mice:

Female CFLP mice were given goldthioglucose (0.85 mg/g body wt., i.p.) and after 17 weeks the obese mice (mean wt. 57g) were selected and randomized into two groups of 4. The compound (20 mg/kg, p.o.) or water was given once daily for 28 days and the mice were killed the day after the last dose. Results are means of 4 values ± S.E.

| | Serum triglyceride (mg/100 ml) | Serum cholesterol (mg/100 ml) |
|---|---|---|
| Control | 35±4 | 82±4 |
| Compound P1 | 22±3* | 81±6 |

* $P < 0.05$

Example 3:

Compound P1 provided the following results with High fat diet-induced obese mice:

Female CFLP mice were fed on a high protein, high fat diet. After 13 weeks the obese mice (mean wt. 49g) were selected and randomized into groups of 12. The compound (20 mg/kg, p.o.) or water was given once daily for 28 days and the mice were killed the day after the last dose. Results are means ± S.E. with numbers of determinations in parentheses.

| | Serum triglyceride (mg/100ml) | Serum cholesterol (mg/100ml) |
|---|---|---|
| Control | 73±8 (12) | 118±3 (12) |
| Compound P1 | 34±2**(12) | 103±5*(12) |

* $P < 0.05$, ** $P < 0.01$.

Example 4:

Compound P2 provided the following results with hypercholesterolaemic mice:

Mice were made hypercholesterolaemic by being fed on a high cholesterol-cholic acid diet for seven days and were dosed orally on the sixth and seventh days. Low density and very low density lipoprotein (LDL and VLDL) cholesterol were measured together by heparin precipitation.

| Total dose on days 6 and 7 (mg/kg) | % Reduction | |
|---|---|---|
| | Total cholesterol | LDL + VLDL cholesterol |
| 25 | 34 | 55 |
| 10 | 22 | 28 |
| 5 | 24 | 29 |

Example 5:

Compound P3 provided the following results with rats:

Male rats were fed on a normal diet or on a diet that contained 0.1g of the compound per kg of diet for 7 days. Results are means of 8 values ± S.E.

| | Serum triglyceride (mg/100ml) | Serum cholesterol (mg/100ml) |
|---|---|---|
| Control | 112±10 | 52±2 |
| Compound P3 | 70±4* | 54±3 |

* $P < 0.05$.

Example 6:

Clinical studies using compound P3:

First study Obese subjects (at least 20% above ideal weight) were given a low energy liquid formula diet and treated with the compound (50 mg pfb, qds) or placebo for six weeks. Serum triglyceride and cholesterol were measured in the fasted state just prior to the first dose and on the morning after the last dose of compound or placebo. Results are means ± S.E. with the number of subjects in parenthesis.

| | Serum triglyceride | | Serum cholesterol | |
|---|---|---|---|---|
| | Initial(mM)† | % Fall | Initial(mM) | % Fall |
| Placebo | 2.33±0.37 (4) | 21±7 | 6.91±0.51 (6) | 19±5 |
| Compound P3 | 2.05±0.16 (4) | 52±6 | 5.81±0.44 (5) | 21±7 |

† Subjects with initial levels below 1 mM excluded from analysis.

Second study  Obese subjects (at least 20% above ideal weight) were given a low energy high fibre diet and treated with the compound or placebo for six weeks after which they were given the alternative treatment for a further six weeks. For the first week of treatment (weeks 1 or 7) the dose level was 50 mg pfb, qds and after this it was 100 mg qds. Serum lipids were measured in the fasted state just prior to the first dose and after each six week period of dosing. Results are means ± S.E. with the number of subjects in parentheses.

| | Serum triglyceride | | Serum cholesterol | | HDL cholesterol | |
|---|---|---|---|---|---|---|
| | Initial(mM) | % change | Initial(mM) | % change | Initial(mM) | % change |
| 1st 6 weeks | | | | | | |
| Placebo (Group A) | 1.45±0.19(18) | -12±5 | 5.89±0.25(19) | -13±3 | 1.41±0.06(16) | -10±3 |
| Compound P3 (Group B) | 1.57±0.18(15) | -25±5 | 6.10±0.24(15) | -9±3 | 1.50±0.07(15) | 0±4 |
| 2nd 6 weeks | | | | | | |
| Compound P3 (Group B) | 1.21±0.18(15) | -4±8 | 5.30±0.29(15) | +5±3 | 1.30±0.04(15) | +14±3 |
| Placebo (Group A) | 1.09±0.12(14) | +33±10 | 5.52±0.30(14) | +4±3 | 1.48±0.07(15) | -5±4 |

Example 7:

Zucker fa/fa male rats of about 550g were dosed once daily with either water or Compound P3 (7.8μmol/kg) for 39 days. Results are means of 8 values ± S.E.M.

| | Plasma Triglyceride (mM) | Plasma Cholesterol (mM) |
|---|---|---|
| Water | 9.3 ± 0.7 | 3.44 ± 0.10 |
| Compound P3 (7.8μmol/kg) | 5.9 ± 0.9* | 3.07 ± 0.14** |

Significant difference from control, *p < 0.02.
**p < 0.05

0244062

EP

CLAIMS

1. The use of a compound selected from the list consisting of compounds of the hereinbefore defined, formulae: (I), (II), (III), (IV), (V), (VI), (VII), (VIII), (IX), (X), (XI), (XII), (XIII), (XIV), (XV), (XVI), (XVII), (XVIII), (XIX), (XX), (XXI), (XXII), (XXIII), (XXIV), (XXV), (XXVI), (XXVII), (XXVIII), (XXIX) and (XXX); or where appropriate, a pharmaceutically acceptable salt, ester or amide thereof, for the manufacture of a medicament for increasing high-density-lipoprotein (HDL) cholesterol concentration and/or decreasing total cholesterol concentration and/or decreasing triglyceride concentration in human blood serum.

2. A use according to claim 1, comprising a compound of formula (I), or a pharmaceutically acceptable salt, ester or amide thereof.

3. A use according to claim 1, comprising a compound of formula (II), or a pharmaceutically acceptable salt, ester or amide thereof.

4. A use according to claim 1, wherein the compound of formula (I) is a compound of formula (IIIA):

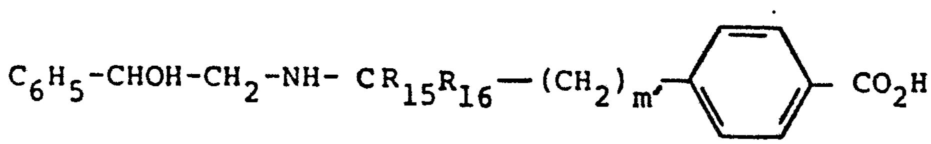

(IIIA)

or a pharmaceutially acceptable salt, ester or amide thereof, wherein $R^{15A}$ is a hydrogen atom or a methyl group, $R^{16A}$ is a hydrogen atom or a methyl group; and m′ is 1,2, or 3

5. A use according to claim 1, wherein, the compound of formula (I) is a compound of formula (IVA):

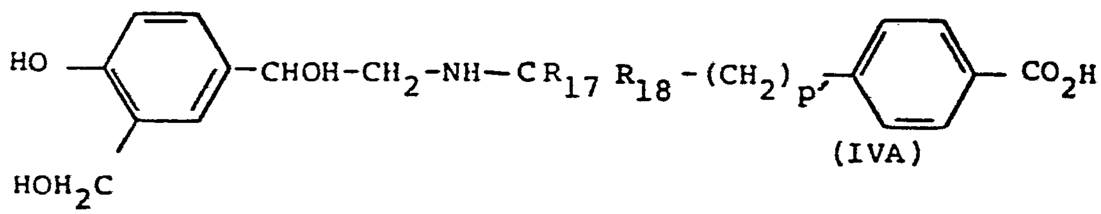

(IVA)

or a pharmaceutically acceptable salt, ester or amide thereof wherein $R^{17A}$ is a hydrogen atom or a methyl group, $R^{18A}$ is a hydrogen atom or a methyl group; and p′ is 1, 2 or 3.

6. A use according to claim 1, wherein the compound of formula (II) is a compound of (VA):

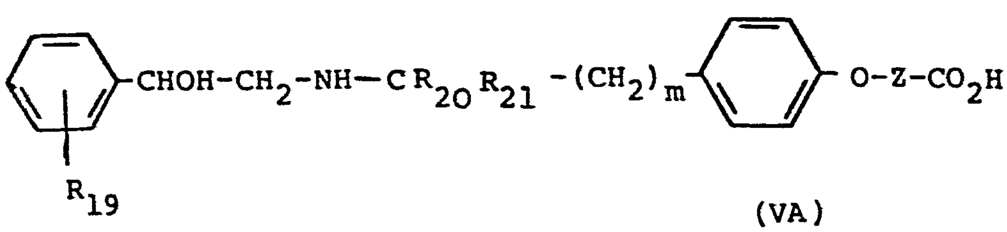

(VA)

or a pharmaceutically acceptable salt, ester or amide thereof, wherein $R^{19}$ is a hydrogen, fluorine or chlorine atom or a trifluoromethyl group, Z is an alkylene, alkenylene or alkyalene group of up to 10 carbon atoms, $R^{20}$ is a hydrogen atom or a methyl group; $R^{21}$ is a hydrogen atom or a methyl group; and m'' is 1, 2, or 3.

7. A use according to claim 1, comprising a compound selected from the list consisting of:

N-[2-(4-carbomethoxyphenyl)-1-methylethyl]-2-hydroxy-2-(4-hydroxy-3-hydroxymethylphenyl)ethanamine;

N-[2-(4-carboxyphenyl)-1-methylethyl]-2-hydroxy-2-(4-hydroxy-3-hydroxymethylphenyl)ethanamine;

N-[2-(4-carboxyphenyl)-1-methylethyl]-2-hydroxy-2-phenylethanamine;

N-[2-(4-carbomethoxyphenyl)-1-methylethyl]-2-hydroxy-2-phenylethanamine;

N-[2-(4-carbomethoxyphenyl)-1-methylethyl]-2-hydroxy-2-(4-amino-3,5-dichlorophenyl)ethanamine;

N-[2-(4-carboxyphenyl)-1-methylethyl]-2-hydroxy-2-(4-amino-3,5-dichlorophenyl)ethanamine;

N-[2-(4-carbomethoxymethoxyphenyl)-1-methylethyl]-2-hydroxy-2-(4-amino-3,5-dichlorophenyl) ethanamine;

N-[2-(4-carboxymethoxyphenyl)-1-methylethyl]-2-hydroxy-2-(4-amino-3,5-dichlorophenyl) ethanamine;

N-[2-(4-carbomethoxymethoxyphenyl)-1-methylethyl]-2-hydroxy-2-(3-chlorophenyl) ethanamine; and

N-[2-(4-carboxymethoxyphenyl)-1-methylethyl]-2-hydroxy-2-(3-chlorophenyl) ethanamine; or a pharmaceutically acceptable acid addition salt thereof.

8. A use according to claim 1, comprising N-[2-(4-carbomethoxyphenyl)-1-methylethyl]-2-hydroxy-2-phenylethanamine; or a pharmaceutically acceptable acid additive salt thereof.

9. A use according to claim 1, comprising N-[2-(4-carbomethoxymethoxyphenyl)-1-methylethyl]-2-hydroxy-2-(3-chlorophenyl) ethanamine; or a pharmaceutically acceptable acid addition salt thereof.